# EUROPEAN PATENT APPLICATION

(11) **EP 2 090 297 A1**
(43) Date of publication of application: **19.08.2009**
(21) Application number: 08101599.2
(22) Date of filing: 13.02.2008
(51) Int. Cl.: A61K 9/16, A61K 9/20, A61K 9/28, A61K 31/496, A61K 9/48, A61K 9/50

(54) **Formulations of flibanserin**

(71) Applicant: Boehringer Ingelheim International GmbH, 55216 Ingelheim am Rhein (DE)
(72) Inventor: The designation of the inventor has not yet been filed
(74) Representative: Hammann, Heinz

(57) **Abstract**

The present invention provides pharmaceutical release systems comprising an therapeutically effective amount of Flibanserin, at least one pharmaceutically acceptable pH modifier and at least one pharmaceutically acceptable excipient achieving and maintaining supersaturation.

## Description

### FIELD OF THE INVENTION

The present invention is directed to pharmaceutical immediate and extended release systems comprising Flibanserin as active ingredient, pharmaceutically acceptable pH modifiers and supersaturizing agents and methods for the production thereof.

### BACKGROUND OF THE INVENTION

Flibanserin is a known benzimidazolon derivative having the summation formula C₂₀H₂₁F₃N₄O represented by the chemical indication 1,3-dihydro-1-[2-[4-[3-(trifluoromethyl)phenyl]-1-piperazinyl]ethyl]-2H-benzimidazole-2-one which was already disclosed in 1992 in form of its hydrochloride in EP-A-526 434 and has the following chemical formula: 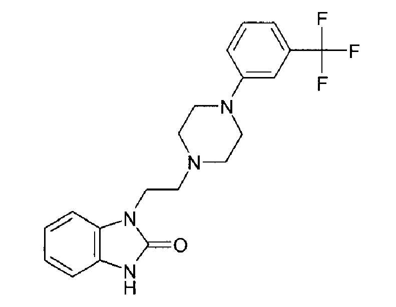 Flibanserin is a known post-synaptic full serotonin (5-HT_{1A}) agonist and 5-HT_{2A} antagonist. It is therefore a promising therapeutic agent for the treatment of a variety of diseases, for instance depression, schizophrenia, and anxiety. Immediate release tablets containing Flibanserin (e.g. as described in WO 03/097058) in conventional solid formulations are well tolerated, but patient compliance could be further improved if a once-daily regimen were possible and if side effects could be even further be reduced. Such a pharmaceutical release system of Flibanserin, which has to be an extended release system, would have not only the advantage of a higher patient compliance but would also be advantageous in having a reduced potential to cause undesirable side effects by reducing the average maximum Flibanserin plasma concentration Cₘₐₓ.

Flibanserin shows a solubility of 6.2 mg/ml in 0.1 N HCl and a solubility of 0.002 mg/ml in 0.05 M phosphate buffer pH 6.8. This marginal solubility at higher pH-values of Flibanserin makes it difficult to develop extended release dosage forms or in some case even immediate release dosage forms for the following reasons. There is a natural pH gradient from the acidity of the stomach where the pH of physiological fluids are typically around 1-2, through the weakly acidic duodenum to the virtually neutral environment of the small intestine where the pH is in the range of 5-8. There are however cases of non-acidic stomach conditions due to either low production of hydrochloric acid caused by the physiology of the patients (this percentage increases form about 2 - 10 % in young patients to about 10 to 40 % in elderly patients) or by coadministration of antacids such as basic agents, H2-blockers or proton pump inhibitors. In those cases dissolution of even immediate release formulations (IRF) may be incomplete in the stomach and absorption might be incomplete or at least delayed.

Dissolution of the active ingredient is a prerequisite for absorption after oral intake. Therefore most suitable formulations for drugs which are poorly soluble at least under certain physiologic pH conditions, are formulations which ensure dissolution independent from the physiologic pH values in the gastrointestinal tract (GIT) tract and maintain the dissolved state in the GIT long enough to allow complete absorption before precipitation occurs. For immediate release preparations it is therefore relevant, that dissolution at pH values ranging from 1 to 6 occurs and supersaturation of the complete dose in a volume of 200 to 250 ml maintains. For extended release formulations dissolution of the entire GIT, that means up to pH 7.5 should occur but only of smaller amounts as the release lasts for hours and volumes are the physiological volumes in the GIT.

It is therefore an object of the present invention to provide improved pharmaceutical immediate and extended release systems which provide a pH-independent release profile in order to improve the bioavailability of Flibanserin. Furthermore methods of manufacturing the same shall be provided.

### DESCRIPTION OF THE INVENTION

It was surprisingly found that by combining a therapeutically effective amount of Flibanserin, with at least one pharmaceutically acceptable pH modifier and at least one pharmaceutically acceptable supersaturizing excipient, a supersaturated status of Flibanserin at higher pH values as present in the gastrointestinal tract can be achieved and maintained

Within the meaning of the present invention, supersaturation occurs if the active ingredient remains in dissolved status at concentrations far above its saturation solubility in the same aqueous system. A supersaturation factor can be calculated as ratio of obtained solubility (in the dissolution system when a formulation according to the invention is used) over the solubility of the active ingredient in buffer at the same pH, (e.g. when dose is 100 mg, buffer volume is 200 ml and 80 % are dissolved in phosphate buffer at pH 5.0, dissolved amount is 100*0.8/200 = 0.4 mg/ml; as solubility of flibanserin in phosphate buffer at pH 5.0 is 0.01 mg/ml, supersaturation factor is 0.4/0.01 = 40).

It has been surprisingly found that the use of certain types of methylcelluloses and certain types of hydroxypropyl methylcelluloses as supersaturizing excipients in proper combination with pharmaceutically acceptable pH modifiers, at least partially levels out the effect of the decreasing solubility of Flibanserin in the lower parts of the GITwhile maintaining sufficiently slow release in the stomach. Further, enhancement of drug release such as Flibanserin in release media of elevated pH is by the addition of pH modifier(s) which create an acidic pH in the micro-environment either within or in the "unstirred" layer around the pharmaceutical formulation and thus improves the solubility of the drug. As a result, the difficulty to establish a suitable balance between the different parts of the GIT with different pH environment has been surprisingly managed.

Therefore, in one embodiment the present invention provides a pharmaceutical formulation for immediate (immediate release system) or extended release (extended release system) comprising or essentially consisting of
I) a core comprising
   a) Flibanserin or a pharmaceutically acceptable derivative thereof as active ingredient;
   b) one or more supersaturizing excipient(s) selected from the group consisting of methylcelluloses, hypromellose 2208, hypromellose 2910 and hypromellose 2906;
   c) one or more pharmaceutically acceptable pH modifiers, wherein the pH modifiers are present in an amount of 45 - 90 % by weight of the core;
   d) optionally one or more additives; and in case of an extended release system
   e) optionally one or more retarding agents; and
   in case of an extended release system, the above described formulation may optionally comprise
II) one more retard layers comprising one or more retarding agents surrounding, but not necessarily in direct contact with the active ingredient.

In a further embodiment the present invention provides a pharmaceutical formulation for immediate (immediate release system) or extended release (extended release system) comprising or essentially consisting of
I) a core comprising
   a) Flibanserin or a pharmaceutically acceptable derivative thereof as active ingredient;
   b) one or more supersaturizing excipient(s) selected from the group consisting of methylcelluloses with nominal viscosity of 15 cP, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP;
   c) one or more pharmaceutically acceptable pH modifiers in a weight ratio of Flibanserin : pH modifiers of 2 : 1 or lower;
   d) optionally one or more additives; and in case of an extended release system
   e) optionally one or more retarding agents; and
   in case of an extended release system, the above described formulation may optionally comprise
II) one more retard layers comprising one or more retarding agents surrounding, but not necessarily in direct contact with the active ingredient.

In a further embodiment the present invention provides for a pharmaceutical formulation for immediate (immediate release system) or extended release (extended release system) comprising or essentially consisting of
I) a core comprising
   a) Flibanserin or a pharmaceutically acceptable derivative thereof as active ingredient;
   b) one or more supersaturizing excipient(s) selected from the group consisting of methylcelluloses with nominal viscosity of 15 cP, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, , hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP;
   c) one or more pharmaceutically acceptable pH modifiers, wherein the pH modifiers are present in an amount of 45 - 90 % by weight of the core;
   d) optionally one or more additives; and in case of an extended release system
   e) optionally one or more retarding agents; and
   in case of an extended release system, the above described formulation may optionally comprise
II) one more retard layers comprising one or more retarding agents surrounding, but not necessarily in direct contact with the active ingredient.

Accordingly, if the pharmaceutical formulations of the present invention are designed as extended release systems, the formulation, in addition to the active ingredient, the supersaturizing agent, the pH modifier and the optional other additives as defined below, may comprise one or more retarding agents in the core (e.g. uniformly distributed as in a matrix tablet; or as a separate layer, which is not surrounding the whole amount of active ingredient) and/or one more retard layers comprising one or more retarding agents, layered around the core achieving extended release of the active ingredient.

Besides these functional excipients steering the desired release profile of Flibanserin in the pharmaceutical immediate and extended release systems according to the present invention, further additives may be optionally comprised in the formulation such as lubricants, binders, fillers, taste masking film coatings, sweeteners etc.

However, surprisingly it has been found, that if the pharmaceutical formulations are produced according to one of the extrusion methods described below, it is possible to produce said formulations without any non functional excipients (with regard to achieve and maintain dissolution of the active ingredient) except a minimal amount of magnesiumstearate or another glidant.

It is therefore provided an immediate or extended release system, particularly for oral administration of Flibanserin which guarantees largely adequate bioavailability of the active ingredient independent form the physiologic pH conditions of the gastrointestinal tract. Therefore, the immediate or extended release formulations of Flibanserin of the present invention provide a near to pH-independent drug release behavior.

The term "system" as used for the expression "immediate release system" or "extended release system" according to the present invention should be understood in its broadest meaning comprising any type of formulation, preparation or pharmaceutical dosage form, which is particularly suitable for oral administration. The immediate or extended release system may be in form of a pellet (derived either from pellet layering or extrusion), tablet, matrix tablet, multilayer tablet, mini tablet, hard or liquid filled capsule. The system may be administered directly, e.g. in form of a tablet, or may be filled in another dosage form such as a capsule. The extended release system according to the present invention is preferably provided in form of a tablet or a multiparticulate system.

In the context of the present invention the term "extended release" should be understood in contrast to "immediate release". The active ingredient is gradually, continuously liberated over time, sometimes slower or faster, but virtually independent from the pH value. In particular, the term indicates that the system does not release the full dose of the active ingredient immediately after oral dosing and that the formulation allows a reduction in dosing frequency.

The term "one or more" or "at least one" as used in the present invention stands, if not otherwise specified, for 1, 2, 3, 4, 5, 6, 7, 8, 9, 10 compounds or even more. Preferred embodiments comprise 1, 2 or 3 such compounds. More preferred embodiments comprise 1 or 2 such compounds and even more preferred are embodiments comprising one of such compounds.

Within the meaning of the present invention, and unless otherwise mentioned, percentage values are to be interpreted as percent by weight and are related to the weight of the core. However, if the term "by weight of the final formulation" is used, percentage values are to be interpreted as percent by weight of the final composition including core and the one more retard layers comprising one or more retarding agents surrounding, but not necessarily in direct contact with the active ingredient mentioned under II) above.

### Pharmaceutically active ingredient (API)

The pharmaceutically active ingredient which is contained in the immediate and extended release system of the present invention is Flibanserin. Flibanserin can be used in form of the free base, or in form of any known pharmacologically acceptable derivative thereof such as its pharmaceutically acceptable acid addition salts and/or optionally in form of the hydrates and/or solvates thereof. Suitable acid addition salts include for example those of the acids selected from succinic acid, hydrobromic acid, acetic acid, fumaric acid, maleic acid, methanesulphonic acid, lactic acid, phosphoric acid, hydrochloric acid, sulphuric acid, tartaric acid and citric acid. Mixtures of the abovementioned acid addition salts may also be used. From the aforementioned acid addition salts the hydrochloride and the hydrobromide, particularly the hydrochloride, are preferred.

If Flibanserin is used in form of the free base, it is preferably used in form of Flibanserin polymorph A which represents the free base of Flibanserin in a specific polymorphic form. Polymorph A and a process for its preparation are disclosed in WO 03/014079 A1, the whole disclosure thereof being incorporated by reference into the present specification.

Flibanserin is contained in an amount suitable for exhibiting the desired pharmacological activities of each medicament, which are known and varies in accordance with the type of medication. Flibanserin is preferably present in a pharmaceutically effective amount (0.01 mg to 200 mg, preferably from 0,1 to 150 mg, from 0,1 to 150 mg or from 0.1 to 50 mg), which, however, may depend from a number of factors for example the age and body weight of the patient, and the nature and stage of the disease. This is deemed to be within the capabilities of the skilled man, and the existing literature on the components can be consulted in order to arrive at the optimum dose. The dosage range applicable per day is between 0.1 to 400, preferably between 1.0 to 300, more preferably between 2 to 200 mg and even more preferably between 3 and 150 mg.

The pharmaceutical release systems of the present invention are administered to the patient preferably once or twice daily. Further, the pharmaceutical release systems of the present invention are administered to the patient preferably once daily in the evening. However, if necessary the formulations of the invention may be administered either two or more times daily consecutively over a period of time.

In the pharmaceutical release system of the present invention the Flibanserin content is preferably in an amount of 10 to 50 %, more preferably in an amount of 20 to 35 %, most preferably in an amount of 24 to 32 % by weight of the core.

The doses given above expressly include all the numerical values, both whole numbers and fractions, within the range specified.

The Flibanserin containing formulation according to the present invention can be used in the treatment of patients suffering from central nervous system disorders, in particular in affective disorders (e.g. depression like major depressive disorder, childhood depression, dysthymia, seasonal affective disorder, dysthymic disorder and minor depressive disorder; bipolar disorders), anxiety (incl. panic disorder with or without agoraphobia, agoraphobia without history of panic disorder, specific phobia (simple phobia), social phobia (social anxiety disorder), obsessive-compulsive disorder (OCD), post-traumatic stress disorder, acute stress disorder, generalized anxiety disorder and anxiety disorder not otherwise specified), sleep and sexual disorders (e.g. Hypoactive Sexual Desire Disorder, premenstrual disorders like premenstrual dysphoria, premenstrual syndrome, premenstrual dysphoric disorder; sexual aversion disorder, sexual arousal disorder, orgasmic disorder, sexual pain disorders like dyspareunia, vaginismus, noncoital sexual pain disorder; sexual dysfunction due to a general medical condition and substance-induced sexual dysfunction), psychosis, schizophrenia (including the disorganized type, the catatonic type, the paranoid type, the undifferentiated type, the residual type of schizophrenia, schizoaffective disorder, schizophreniform disorder, delusional disorder, brief psychotic disorder, shared psychotic disorder, psychotic disorder due to a general medical condition, substance-induced psychotic disorder, and psychotic disorder not otherwise specified), personality disorders, mental organic disorders, mental disorders in childhood, aggressiveness, age associated memory impairment, for neuroprotection, the treatment and/or prevention of neurodegenerative diseases as well as cerebral ischaemia of various origins (e.g. epilepsy, hypoglycaemia, hypoxia, anoxia, brain trauma, brain oedema, amyotropic lateral sclerosis, Huntington's disease, Alzheimer's disease, hypotension, cardiac infarct, brain pressure (elevated intracranial pressure), ischaemic and haemorrhagic stroke (stroke), global cerebral ischaemia during stoppage of the heart, diabetic polyneuropathy, tinnitus, perinatal asphyxia, cardiac hypertrophia (thickening of the heart muscle) and cardiac insufficiency (weakness of the heart muscle); anorexia nervosa (incl. binge-eating/purging type of anorexia nervosa and the restricting type of anorexia nervosa), Attention Deficit Hyperactivity Disorder (ADHD) (incl. ADHD predominantly combined type, ADHD predominantly inattentive type, and ADHD predominantly hyperactive-impulsive type), obesity (incl. exogenic obesity, hyperinsulinaemic obesity, hyperplasmic obesity, hyperphyseal adiposity, hypoplasmic obesity, hypothyroid obesity, hypothalamic obesity, symptomatic obesity, infantile obesity, upper body obesity, alimentary obesity, hypogonadal obesity and central obesity), urinary incontinence (incl. overactive bladder syndrome, urgency, urge urinary incontinence, stress urinary incontinence, mixed urinary incontinence), chronic pain (incl. neuropathic pain, diabetic neuropathy, post-herpetic neuralgia (PHN), carpal tunnel syndrome (CTS), HIV neuropathy, phantom limb pain, complex regional pain syndrome (CPRS), trigeminal neuralgia / trigeminus neuralgia / tic douloureux, surgical intervention (e.g. postoperative analgesics), diabetic vasculopathy, capillary resistance or diabetic symptoms associated with insulitis, pain associated with angina, pain associated with menstruation, pain associated with cancer, dental pain, headache, migraine, trigeminal neuralgia, temporomandibular joint syndrome, myofascial pain muscular injury, fibromyalgia syndrome, bone and joint pain (osteoarthritis), rheumatoid arthritis, rheumatoid arthritis and edema resulting from trauma associated with burns, sprains or fracture bone pain due to osteoarthritis, osteoporosis, bone metastases or unknown reasons, gout, fibrositis, myofascial pain, thoracic outlet syndromes, upper back pain or lower back pain (wherein the back pain results from systematic, regional, or primary spine disease (radiculopathy), pelvic pain, cardiac chest pain, non-cardiac chest pain, spinal cord injury (SCI)-associated pain, central post-stroke pain, cancer neuropathy, AIDS pain, sickle cell pain and geriatric pain), Valvular Heart Disease (incl. valvular stenosis, valvular regurgitation, atresia of one of the valves, mitral valve prolapse), preferably Hypoactive Sexual Desire Disorder (HSDD).

Accordingly, in another aspect the present invention comprises a pharmaceutical formulation or a pharmaceutical release system as described hereinbefore and below for use in a method of therapy of the human or non-human animal body, preferably in a method of treating of the above mentioned diseases and conditions.

This invention also relates to a pharmaceutical composition, a pharmaceutical formulation or a pharmaceutical release system as described herein before and below for use in the manufacture of a pharmaceutical dosage form, preferably for oral administration to a mammal in need of treatment, preferably for the treatment of the above mentioned diseases and conditions.

### PH modifiers as functional excipients (A)

According to the present invention the term "pH modifiers" is not limited to organic acids but any pharmaceutical acceptable chemical substance capable of providing an acidic pH value may be used. Usually the pH modifiers may be selected from one or more pharmacologically acceptable organic or inorganic acids and/or buffers or mixtures thereof. However, the use of organic acids is preferred.

The pharmaceutically acceptable organic acids may be preferably selected from the group consisting of acetic acid, adipic acid, ascorbic acid, asparagines, aspartic acid, benzenesulphonic acid (besylate), benzoic acid, p-bromophenylsulphonic acid, camphorsulphonic acid, carbonic acid, gamma-carboxyglutamic acid, citric acid, cysteine, ethanesulphonic acid, fumaric acid, particularly cis-fumaric acid and/or trans-fumaric acid, gluconic acid, glutamic acid, glutaric acid, I-glutamine, hydrobromic acid, hydrochloric acid, hydroiodic acid, isethionic acid, isoleucine, lactic acid, I-leucine, maleic acid, malic acid, malonic acid, mandelic acid, methanesulphonic acid (mesylate), methionine, mucinic acid, nitric acid, omithine, oxalic acid, pamoic acid, pantothenic acid, phosphoric acid, serine, succinic acid, sulphuric acid, tartaric acid, p-toluenesulphonic acid, tyrosine glutamic acid, valine and derivatives and mixtures thereof. The above listing is not intended to be of limitative character, the skilled person is familiar with further examples.
Preferred are adipic acid, ascorbic acid, aspartic acid, citric acid, fumaric acid, lactic acid, malic acid, succinic acid and tartaric acid, more preferred are succinic acid, tartaric acid, lactic acid and fumaric acid.
Surprisingly it has been found that the use of a combination of tartaric acid and lactic acid, or a combination of tartaric acid and fumaric acid or a combination of tartaric acid, lactic acid and fumaric acid have the advantage of improved dissolution, and show in addition a slightly retarding effect, of formulations so that in some cases the use of other retarding agents is not necessary. Lactic acid acts additionally as a plasticizer if melt extrusion is employed

Accordingly, especially preferred are those embodiments that comprise a combination of tartaric acid and lactic acid, or a combination of tartaric acid and fumaric acid or a combination of tartaric acid, lactic acid and fumaric acid.

The pH modifier(s) is (are) preferably present in an amount of 45 - 90 %, more preferably 50 - 80 %, most preferably 55 - 77 %, particularly 58 - 72 % by weight of the core.

It should be noted that the ranges of values given herein expressly include all the numerical values, both whole numbers and fractions, within the ranges as specified.

### Supersaturizing excipients (SSE)

Even if dissolution of the API is achieved by addition of pH modifiers, adequate absorption is not obtained if the API precipitates after stomach emptying at the higher pH values of about 5 to 6.5 in the upper GI tract. Surprisingly it has been found that cellulose derivatives such as methylcellulose and hydroxypropyl methylcelluloses, which are well known as binders, film forming agents, retarding agents etc. are suitable to achieve and maintain supersaturation of Flibanserin which was not yet known before.

Methylcelluloses and hydroxypropyl methylcelluloses (e.g. available under the tradename Methocel) are available in a great variety of different types with different grades and types of substitutions resulting in different viscosities. For example, Methylcellulose, which is a methyl ether of Cellulose, is available in different grades having different viscosities e.g. of 15, 400, 1500 and 4000 cP. Hydroxy propylmethylcellulose (HPMC) is a propylene glycol ether of methylcellulose available in different substitution types. For example, for HPMC type 1828 the content of methoxy groups according to USP has to be between 16.5 and 20 % and the content of the hydroxypropoxy groups has to be between 23 and 32 % (after drying at 105 °C for 2 h). For HPMC type 2208 the content of methoxy groups according to USP II has to be between 19 and 24 % and the content of the hydroxypropoxy groups has to be between 4 and 12 %. For HPMC type 2906 the content of methoxy groups according to USP has to be between 27 and 30 % and the content of the hydroxypropoxy groups has to be between 4 and 7.5 %. For HPMC type 2910 the content of methoxy groups according to USP has to be between 28 and 30 % and the content of the hydroxypropoxy groups has to be between 7 and 12 %. All of those varieties of HPMC are available with different nominal viscosities varying between 3 and 100000 cP (2% solution in water; w/v, at 20 °C)

The supersaturizing excipients of the immediate or extended release systems of the present invention which can be used in combination with Flibanserin in order to achieve a pH-independent release are one or more methylcelluloses and/or one or more hydroxypropyl methylcelluloses selected from the group consisting of hypromellose 2208, hypromellose 2910, hypromellose 2906

In a further aspect of the present invention the supersaturizing excipients of the immediate or extended release systems of the present invention which can be used in combination with Flibanserin in order to achieve a pH-independent release are one or more methylcelluloses and/or one or more hydroxypropyl methylcelluloses selected from the group consisting of methylcelluloses with nominal viscosity of 15 cP, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP.

In another aspect of the present invention the supersaturizing excipients of the immediate or extended release systems of the present invention which can be used in combination with Flibanserin in order to achieve a pH-independent release are one or more methylcelluloses and/or one or more hydroxypropyl methylcelluloses selected from the group consisting of methylcelluloses with nominal viscosity of 15 cP, methylcelluloses with nominal viscosity of 400 cP, hypromellose 2910 with nominal viscosity of 5 cP, hypromellose 2910 with nominal viscosity of 6 cP and hypromellose 2906 with nominal viscosity of 50 cP and hypromellose 2906 with nominal viscosity of 4000 cP.

The term "nominal viscosity" as used according to the present invention in the term "hypromellose 2208 with nominal viscosity of 4000 cP" embraces hypromellose with a viscosity range around 4000 cP (as 2% solution in water; w/v at 20 °C) as defined by USP II/NF, which is in this case 3000-5600 cP (as 2% solution in water; w/v at 20 °C). For the other hypromelloses and methylcelluloses according to the present the corresponding ranges apply.

The supersaturizing excipient(s) is (are) preferably present in an amount of 0.3 - 40 %, more preferably 0.6 - 20 %, most preferably 1 - 15 %, particularly 2 - 10 % by weight of the core.

### pH independent Polymers as retarding agents (I-ER)

The pH-independent polymer of the immediate or extended release systems is not limited according to the present invention; it may be used any pharmaceutically acceptable polymer which has a solubility characteristic being independent from the pH value of the environment.

The one or more pH-independent polymers of the present invention comprise alkylcelluloses, such as, methylcellulose, ethylcelluloses; hydroxyalkyl celluloses, for example, hydroxymethyl cellulose, hydroxyethyl cellulose, hydroxypropyl cellulose and hydroxybutyl cellulose; hydroxyalkyl alkylcelluloses, such as hydroxyethyl methylcellulose and hydroxypropyl methylcellulose; carboxyalkylcellulose esters; other natural, semi-synthetic, or synthetic di-, oligo- and polysaccharides such as galactomannans, tragacanth, agar, guar gum, and polyfructans; ammonio methacrylate copolymers; polyvinylalcohol; polyvinylpyrrolidone, copolymers of polyvinylpyrrolidone with vinyl acetate; combinations of polyvinylalcohol and polyvinylpyrrolidone; polyalkylene oxides such as polyethylene oxide and polypropylene oxide; copolymers of ethylene oxide and propylene oxide as well as derivatives and mixtures thereof; preferably cellulose ether derivatives such as hydroxypropyl methylcellulose and hydroxypropyl cellulose, most preferred hydroxypropyl methylcellulose, for example Methocel ethers. In this case, if hypromelloses and methylcelluloses according to the present invention are used, those may function not only as supersaturizer but also as pH independent polymer.

The term "derivatives" according to the present invention is meant to include any compound derived from the mentioned compounds as basic system, for example by substitution with one or more functional groups. This belongs to the general knowledge of the skilled person.

The pH-independent polymer may be used alone or in combination of two or more pH-independent polymers or may be combined with pH-dependent polymers. The pH-independent polymer(s) may be present in an amount of 0.5 - 50 %, preferably 1 - 30 %, more preferably 2 - 15 % and most preferably 2.5 - 10 % by weight of the final formulation.

### pH dependent polymers as retarding agents (D-ER)

Also the pH-dependent polymer of the extended release systems is not limited according to the present invention. Any pharmaceutically acceptable polymer may be used which has a pH-dependent solubility, preferably a polymer which has a high solubility in high pH medium and a low solubility in low pH medium in the sense that the solubility of the polymer is preferably better in high pH medium (pH about more than 4) compared with low pH medium (pH about 1-2).

The pH-dependent polymer(s) of the present invention comprises acrylic acid polymerisate, methacrylic acid copolymers, alginates, carrageenans, acacia, xanthan gum, chitin derivates such as chitosan, carmellose sodium, carmellose calcium, phthalate such as hydroxypropyl methyl cellulose phthalate, cellulose acetate phthalate, polyvinyl acetate phthalate, trimellitate such as cellulose acetate trimellitate, shellac and derivatives and mixtures thereof, preferably methacrylic acid copolymers such as poly(methacrylic acid, ethylacrylate) 1:1 (Eudragit^{®} L 100-55), poly(methacrylic acid, methyl methacrylate) 1:1 (Eudragit^{®} L 100), poly(methacrylic acid, methyl methacrylate) 1:2 (Eudragit^{®} S), and alginates (such as Protanal^{®}), most preferably used are Eudragit^{®} L and Protanal^{®}.

The pH-dependent polymer may be used alone or in combination of two or more pH-dependent and/or one or more pH-independent polymers. The pH-dependent and pH-independent polymer(s) may be present in an amount of 0.1 - 25 %, more preferably 0.25 - 15 %, most preferably 0.5 - 10 %, particularly 0.6 - 8 % by weight of the final formulation.

In one embodiment the extended release system according to the present invention the term "one or more" or "at least one" as used in the present invention stands for 1, 2, 3, 4, 5, 6, 7, 8, 9, 10 compounds or even more. Preferred embodiments comprises 1 or a mixture of 2, or 3 D-ER's and/or I-ER's . In another embodiment the extended release systems according to the present invention comprise a mixture of 2, D-ER's and/or I-ER's.

The selection of polymers for the extended release systems of the present invention, at least one pH-dependent and either no or at least one pH-independent, have an influence on the release of the Flibanserin in order to establish the desired release profiles. Although the active substance presented has a pH-dependent solubility the release profile of the extended release system according to the present invention is either almost independent from the pH value or release becomes even faster at higher pH values in order to compensate for the diminished solubility and the decreasing absorbability in the lower gastrointestinal tract, resulting in an improved bioavailability.

The aforementioned immediate and extended release systems of the present invention may also comprise optionally one or more additives e. g. carriers, technological adjuvants such as lubricants, glidants, granulating agents, anti-caking agents, agglomeration inhibitors, antiadherents, anti-tacking agent, anti-sticking agent, flavors, aromatiziers, dyes or colorants, preservatives, plastizers, wetting agents, sweeteners, chelating agents, stabilizers, solubilizers, antioxidants, fillers, diluents and the like. These pharmaceutically acceptable formulating agents are e.g. present in order to promote the manufacture, compressibility, appearance and/or taste of the preparation. Other conventional additives known in the art can also be included. The above listing is not intended to be of limitative character, the skilled person is familiar with further examples.

### Lubricants (L)

A lubricant or agglomeration inhibitor can be used to enhance release of the dosage form from the apparatus on which it is formed, for example by preventing adherence to the surface of an upper punch ("picking") or lower punch ("sticking"). These materials may also possess antiadherent or glidant properties. Preferable lubricants are for example stearic acid as well as salts thereof including sodium stearate, calcium stearate, zinc stearate, magnesium stearate, glyceryl monostearate, particularly magnesium stearate, polyethylene glycols (all types at different molecular weights of PEGs), fumaric acid, glycerides such as glyceryl behenate (Compritol^{®} 888), Dynasan^{®} 118 or Boeson^{®} VP.

### Anti-sticking (glidant) agents (G)

An anti-tacking agent, anti-sticking agent or glidant or an agent to improve flowability can be used to improve powder flow properties prior to and during the manufacturing process and to reduce caking. Among this group of excipients may be exemplarily mentioned silicon dioxide, particularly colloidal silicon dioxide (e.g. Aerosil^{®}, Cab-O-Sil^{®}), stearic acid as well as salts thereof including sodium stearate, calcium stearate, zinc stearate, magnesium stearate, magnesium silicate, calcium silicate, magnesium trisilicate and talc. Preferably glidants are colloidal silicon dioxide and talc.

### Binders (B)

As binder, it is possible to use any binder usually employed in pharmaceuticals. Exemplarily mentioned are naturally occurring or partially or totally synthetic polymers selected from acacia, agar, alginic acid, carbomers, carmellose sodium, carrageenan, cellulose acetate phthalate, ceratonia, chitosan, confectionar's sugar, copovidone, povidone, cottonseed oil, dextrate, dextrin, dextrose, polydextrose, maltodextrin, maltose, cellulose and derivatives thereof such as microcrystalline cellulose, ethylcellulose, hydroxyethyl cellulose, hydroxyethyl methylcellulose, hydroxypropyl celluloses, carboxymethylcelluloses, starch and derivatives thereof, such as pregelatinized starch, hydroxypropylstarch, corn starch, gelatin, glyceryl behenate, tragacanth, guar gum, hydrogenated vegetable oils, inulin, lactose, glucose, magnesium aluminium silicate, poloxamer, polycarbophils, polyethylene oxide, polyvinylpyrrolidone, copolymers of N-vinylpyrrolidone and vinyl acetate, polymethacrylates, polyethylene glycols, alginates such as sodium alginate, gelatin, sucrose, sunflower oil, zein as well as derivatives and mixtures thereof.

Particularly preferred binders are acacia, hydroxypropyl celluloses, hydroxypropyl methylcelluloses, methylcelluloses, hydroxyethyl celluloses, carboxymethylcelluloses, polyvinylpyrrolidone, the copolymers of N-vinylpyrrolidone and vinyl acetate, or combinations of these polymers. The above listing is not intended to be of limitative character, the skilled person is familiar with further examples.

As further additives which may be present the following non limitative groups are given

### Preservatives (P)

- preservatives, preferably antimicrobial preservatives such as benzalkonium chloride, benzoic acid, methyl parahydroxybenzoate, propyl parahydroxybenzoate, sodium benzoate and sorbic acid;

### Sweetening agents (SW)

- sweetening agents such as acesulfame potassium, alitame, aspartame, compressible sugar, confectioner's sugar, dextrose, erythritol, fructose, glycerin, inulin, isomalt, lactitol, liquid glucose, maltitol, maltitol solution, maltose, mannitol, neospheridin dihydrochalcone, polydextrose, saccharin, saccharin sodium, sodium cyclamate, sorbitol, sucralose, sucrose, thaumatin, trehalose, xylitol;

### Meltable excipients used for melt extrusion (ME)

Suitable are excipients having melting points or softening temperatures below 150°C. Especially preferred however are excipients like polyethylenglykoles (PEG) and polyethyleneoxides (POE) with molecular weights from 1000 to 7000000, poloxamer types, medium to long chain fatty alcohols and acids and derivatives thereof, waxes like Cutina HR or Carnaubawax. The PEG derivatives with MW > 100000 and the waxes may serve as retarding agents additionally.

### Separating agents (SA)

- separating agents such as e.g. talc, magnesium stearate or silicic acid serves to prevent the particles from aggregating during the manufacturing process;

### Plasticizers (PL)

- plasticizers are preferably not present in the extended release system which is usually free of plasticizer; however in some rare cases the plasticizers may be selected from e.g. citrates such as acetyltributyl citrate, acetyltriethyl citrate, tributyl citrate, triethyl citrate, benzyl benzoate, castor oil, phthalates such as cellulose acetate phthalate, dibutyl phthalate, diethyl phthalate, dimethyl phthalate, hypromellose phthalate, polyvinyl acetate phthalate, dimeticon, fractionated coconut oil, chlorbutanol, dextrin, sebacate such as dibutyl sebacate, glycerin, glycerin derivatives such as glycerol monostearate, glycerol triacetate (triacetin), acetylated monoglyceride, mannitol, mineral oil, lanolin alcohols, palimitic acid, 2-pyrrolidone, sorbitol, stearic acid, triethanolamin, polyethyleneglycols (all types at different molecular weights of PEGs and POEs), and propylene glycol, and derivatives and mixtures thereof,

### Pigments (PI)

- pigments which are especially useful are titanium dioxide, indigo carmine, iron oxide pigments such as iron oxides red and yellow, and some of the aluminium lakes as well as pigment black, pigment white, pigment yellow, sunset yellow, sunset yellow lake, quinoline yellow lake and the like.

The extended release systems of the present inventions additionally comprise one or more excipient(s) with diluting or filling properties (fillers or diluents). Fillers or diluents are inert compounds designed to make up the required bulk of the dosage form when the drug dosage itself is inadequate to produce this bulk.

### Fillers and/or diluents (FD)

Suitable fillers or diluents may be selected from, for example, lactose, in particular lactose monohydrate, talc, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, cellulose and derivatives, such as powdered cellulose, microcrystalline or silicified microcrystalline cellulose, cellulose acetate, sugars and derivatives such as confectioner's sugar, fructose, sucrose, dextrates, dextrin, D-sorbitol sulfobutylether β-cyclodextrin, dextrose, polydextrose, trehalose, maltose, maltitol, mannitol, maltodextrin, sorbitol, inulin, xylitol, erythritol, isomalt, kaolin and lactitol.

### Chelating agents (CH)

Possible chelating agents which may be added are edetic acid, dipotassium edetate, disodium edetate, edetate calcium disoidium, trisodium edetate, maltol and the like.

It is a matter of course that an additive may have more than one functionality so that they may be categorized among more than one type of additive. For example corn starch or pregelatinized starch may impart several functions at the same time such as swelling polymer, filler, glidant, and the like. However, the skilled person knows the several functions and is able to select the additive according to the intended use thereof.

### Excipients used for final coating (EFC)

The resulting extended release system may finally be coated with a coating preferably of a pharmaceutically conventional film forming agent, and optionally additives. This may be done by conventional methods. Coating serves to mask the taste of the drug, make e. g. a tablet easier to swallow, to reduce any increased abrasion during packing, e. g. into capsules, to increase the shelf life and/or as further diffusion barrier, in some cases, it may improve the appearance of the dosage form. In several cases however, the extended release coating may be sufficient for the tasks mentioned above.

The extended release system can be sugar coated according to procedures well known in the art, or can be coated with any one of numerous polymeric film-forming agents frequently employed by formulation chemists. Suitable film-forming agents include for example ammonium alginate, chitosan, chlorpheniramine maleate, copovidone, phthalate such as dibutyl phthalate, diethyl phthalate, dimethyl phthalate, cellulose acetate phthalate, polyvinyl acetate phthalate, dibutyl sebacate, ethyl lactate, alkylcelluloses and derivatives thereof such as ethylcelluloses, methylcelluloses, gelatin, hydroxyalkyl celluloses and derivatives thereof such as hydroxyethyl cellulose, hydroxypropyl cellulose, hydroxyalkyl alkylcellulose and derivatives thereof such as hypromelloses (hydroxypropyl methylcellulose), hydroxypropyl methylcellulose acetate succinate, hydroxypropyl methylcellulose phthalate, cellulose acetate trimellitate, cellulose acetate phthalate, maltodextrin, calcium carbonate, polydextrose, polyethylene glycols (all types at different molecular weigths of PEGs), polyethylene oxide, polymers and copolymers of acrylic and methacrylic acid and the esters thereof, or combinations of these polymers such as polymethacrylates, poly(methylvinyl ether/maleic anhydride), polyvinyl acetate phthalate, triethyl citrate, vanillin, shellac as well as derivatives and mixtures thereof.

Particularly preferred film-forming agents are hydroxypropyl cellulose, hydroxypropyl methylcellulose, methylcelluloses, polymers and copolymers of acrylic and methacrylic acid and the esters thereof, or combinations of these polymers. Preferably polymers are poly(methacrylic acid, ethylacrylate) 1:1 (Eudragit^{®} L 100-55 or Eudragit^{®} L 30D-55); poly(methacrylic acid, methyl methacrylate) 1:1 (Eudragit^{®} L 100); poly(methacrylic acid, methyl methacrylate) 1:2 (Eudragit^{®} S); hydroxypropyl methylcellulose acetate succinate, hydroxypropyl methylcellulose phthalate (HPMCP 50 or 55), cellulose acetate trimellitate, cellulose acetate phthalate (Aquacoate^{®} CPD), polyvinyl acetate phthalate (Sureteric^{®}), and shellac.

Further suitable additives, excipients, diluents, carriers, technological adjuvants, if desired, may be present.

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.3 - 40 % |
| one or more pharmaceutically acceptable pH modifiers; | 45 - 90 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.6 - 20 % |
| one or more pharmaceutically acceptable pH modifiers; | 45 - 90 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 1 - 15 % |
| one or more pharmaceutically acceptable pH modifiers; | 45 - 90 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 2 - 10 % |
| one or more pharmaceutically acceptable pH modifiers; | 45 - 90 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.3 - 40 % |
| one or more pharmaceutically acceptable pH modifiers; | 50 - 80 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.6 - 20 % |
| one or more pharmaceutically acceptable pH modifiers; | 50 - 80 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 1 - 15 % |
| one or more pharmaceutically acceptable pH modifiers; | 50 - 80 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 2 - 10 % |
| one or more pharmaceutically acceptable pH modifiers; | 50 - 80 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.3 - 40 % |
| one or more pharmaceutically acceptable pH modifiers; | 55 - 77 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.6 - 20 % |
| one or more pharmaceutically acceptable pH modifiers; | 55 - 77 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 1 - 15 % |
| one or more pharmaceutically acceptable pH modifiers; | 55 - 77 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 2 - 10 % |
| one or more pharmaceutically acceptable pH modifiers; | 55 - 77 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.3 - 40 % |
| one or more pharmaceutically acceptable pH modifiers; | 58 - 72 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.6 - 20 % |
| one or more pharmaceutically acceptable pH modifiers; | 58 - 72 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 1 - 15 % |
| one or more pharmaceutically acceptable pH modifiers; | 58 - 72 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 2 - 10 % |
| one or more pharmaceutically acceptable pH modifiers; | 58 - 72 % |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.3 - 40 % |
| one or more pharmaceutically acceptable pH modifiers in a weight ratio of Flibanserin : pH modifiers of 2 : 1 or lower; | |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 0.6 - 20 % |
| one or more pharmaceutically acceptable pH modifiers in a weight ratio of Flibanserin : pH modifiers of 2 : 1 or lower; | |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 1 - 15 % |
| one or more pharmaceutically acceptable pH modifiers in a weight ratio of Flibanserin : pH modifiers of 2 : 1 or lower; | |
| additional additives | ad 100 % |

According to one embodiment the release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses, hypromellose 2208, hypromellose 2910 and hypromellose 2906; | 2 - 10 % |
| one or more pharmaceutically acceptable pH modifiers in a weight ratio of Flibanserin : pH modifiers of 2 : 1 or lower; | |
| additional additives | ad 100 % |

A further embodiment of the present invention is defined by any of the above mentioned embodiments, wherein the one or more supersaturizing excipients are selected from the group consisting of methylcelluloses with nominal viscosity of 15 cP,methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP.

Further embodiments of the present invention are defined by any of the above mentioned embodiments, comprising either one or more supersaturizing excipients selected from the group consisting of methylcelluloses, hypromellose 2208, hypromellose 2910 and hypromellose 2906, or one or more supersaturizing excipients selected from the group consisting of methylcelluloses with nominal viscosity of 15 cP, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP;, wherein the one or pharmaceutically acceptable pH modifiers are selected from the group consisting of fumaric acid, lactic acid and tartaric acid. Especially preferred are those embodiments that comprise a combination of tartaric acid and lactic acid, or a combination of tartaric acid and fumaric acid or a combination of tartaric acid, lactic acid and fumaric acid.

A further embodiment of the present invention is defined by any of the above mentioned embodiments, wherein the one or more supersaturizing excipients are selected from the group consisting of methylcelluloses with nominal viscosity of 15 cP, methylcelluloses with nominal viscosity of 400 cP, hypromellose 2910 with nominal viscosity of 5 cP, hypromellose 2910 with nominal viscosity of 6 cP and hypromellose 2906 with nominal viscosity of 50 cP and hypromellose 2906 with nominal viscosity of 4000 cP.

Further embodiments of the present invention are defined by any of the above mentioned embodiments, comprising either one or more supersaturizing excipients selected from the group consisting of methylcelluloses with nominal viscosity of 15 cP, methylcelluloses with nominal viscosity of 400 cP, hypromellose 2910 with nominal viscosity of 5 cP, hypromellose 2910 with nominal viscosity of 6 cP and hypromellose 2906 with nominal viscosity of 50 cP and hypromellose 2906 with nominal viscosity of 4000 cP wherein the one or pharmaceutically acceptable pH modifiers are selected from the group consisting of fumaric acid, lactic acid and tartaric acid. Especially preferred are those embodiments that comprise a combination of tartaric acid and lactic acid, or a combination of tartaric acid and fumaric acid or a combination of tartaric acid, lactic acid and fumaric acid.

Accordingly preferred release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses with nominal viscosity of 15 cP, methylcelluloses with nominal viscosity of 400 cP, methylcelluloses | 2 - 10 % |
| with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP. | |
| one or more pharmaceutically acceptable pH modifiers selected from the group consisting of fumaric acid, lactic acid and tartaric acid or combination of tartaric acid and lactic acid, or a combination of tartaric acid and fumaric acid or a combination of tartaric acid, lactic acid and fumaric acid; | 58 - 72 % |
| additional additives | ad 100 % |

Accordingly preferred release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses with nominal viscosity of 15 cP, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP. one or more pharmaceutically acceptable pH modifiers in a weight ratio of Flibanserin : pH modifiers of 2 : 1 or lower; wherein the pH | 2 - 10 % |
| modifier is selected from the group consisting of fumaric acid, lactic acid and tartaric acid or a combination of tartaric acid and lactic acid, or a combination of tartaric acid and fumaric acid or a combination of tartaric acid, lactic acid and fumaric acid; additional additives | ad 100 % |
| | |
| Flibanserin or a pharmaceutically acceptable derivative thereof | 10 - 50 % |
| one or more supersaturizing excipient selected from the group consisting of methylcelluloses with nominal viscosity of 15 cP, methylcelluloses with nominal viscosity of 400 cP, hypromellose 2910 with nominal viscosity of 5 cP, hypromellose 2910 with nominal viscosity of 6 cP and hypromellose 2906 with nominal viscosity of 50 cP and hypromellose 2906 with nominal viscosity of 4000 cP; | 2 - 10 % |
| one or more pharmaceutically acceptable pH modifiers selected from the group consisting of fumaric acid, lactic acid and tartaric acid or combination of tartaric acid and lactic acid, or a combination of tartaric acid and fumaric acid or a combination of tartaric acid, lactic acid and fumaric acid; | 58 - 72 % |
| additional additives | ad 100 % |

Accordingly preferred release systems of the present invention comprise or are consisting of:

| | |
|---|---|
| Flibanserin or a pharmaceutically acceptable derivative thereof | 10 - 50 % |
| one or more supersaturizing excipient selected from the group consisting of methylcelluloses with nominal viscosity of 15 cP, methylcelluloses with nominal viscosity of 400 cP, hypromellose 2910 with nominal viscosity of 5 cP, hypromellose 2910 with nominal viscosity of 6 cP and hypromellose 2906 with nominal viscosity of 50 cP and hypromellose 2906 with nominal viscosity of 4000 cP; one or more pharmaceutically acceptable pH modifiers in a weight ratio of Flibanserin : pH modifiers of 2 : 1 or lower; wherein the pH | 2 - 10 % |
| modifier is selected from the group consisting of fumaric acid, lactic acid and tartaric acid or a combination of tartaric acid and lactic acid, or a combination of tartaric acid and fumaric acid or a combination of tartaric acid, lactic acid and fumaric acid; | |
| additional additives | ad 100 % |

Further embodiments of the present invention relate to any of the above mentioned embodiments that contain in addition to the above list of ingredients one or more pharmaceutically acceptable pH-dependent and/or pH-independent polymers in order to create an extended release of Flibanserin. The said polymers (retarding agents) may be contained in the core (e.g. uniformly distributed as in a matrix tablet; or as a separate layer, (not surrounding the whole amount of active ingredient) and/or in one more retard layers comprising one or more retarding agents, layered around the core achieving extended release of the active ingredient.

The pH-dependent and pH-independent polymer(s) may be present in an amount of 0.1 - 25 % more preferably 0.25 - 15 % most preferably 0.5 - 10 % particularly 0.6 - 8 % by weight of the final formulation.

Preferrably the extended release systems of the present invention comprise a polymer selected from the group consisting of Eudragit^{®} S and Hypromellose-phthalate HP 55 combined with antisticking agent such as talc and pore formers such as triethylcitrate.

The formulations according to the invention may be in any form convenient for topical administration or administration into an externally voiding body cavity (e.g. nose, lungs, mouth, ear, stomach, rectum or vagina). Typical administration forms include but are not limited to tablets, buccal tablets, lozenges, coated tablets, capsules. suppositories, chewing gum, gels, powders, granules, syrups and dispersions, although capsules and tablets are preferred.

Thus another object of the present invention is an orally to take pharmaceutical immediate or extended release system, in particular capsules and tablets, like tablets for swallowing, bilayer tablets, triple layer tablets, floating tablets, sugar-coated tablets, coated tablets, chewable tablets, matrix tablets, pills or capsules.

The formulations of the present invention may be prepared by methods which are well known to those skilled in the art, for example production of tablets by wet granulation, direct compression or roller compaction process can be applied to the manufacturing of the immediate or extended release system. Alternatives are multiparticulates e.g. pellets which are either produced by layering processes, rotor pelletization, pelletization in high shear mixers or extrusion. An also suitable process is spray drying of the active ingredient and the functional excipients which may result in amorphous active ingredients.

If the immediate or extended release system which is subject of the present invention is a tablet, preferably it shall have a round or oval shape. The size thereof preferably shall be between 5 mm and 12 mm diameter in case of round shape and between 6 x 12 mm and 10 x 20 mm in case of oval shape. The weight thereof preferably shall be between 50 and 1000 mg.

The immediate or extended release system of the present invention can be of any suitable size and shape, for example round, oval, polygonal or pillow-shaped, and optionally bear non-functional surface markings.

The immediate or extended release system of the present invention remains sufficiently stable when stored. Only after the administration of the formulation system does the pH modifier dissolve and produce a micro climate in which the active substance can dissolve.

For extended release formulations, the retardation can either be achieved by adding retarding excipients to the composition of the formulation (matrix systems), or the retardation can be achieved by spraying retarding lacquers onto immediate or moderately extended release formulations (diffusion coatings). Even though both mechanisms can be combined either way, preferred alternatives are matrix tablets and multiparticulates with diffusion coatings, whose general methods of manufacture are described below.

### Preparation of tablet matrix systems

As already mentioned above, the present immediate or extended release systems of the present invention may be prepared by methods which are well known to those skilled in the art, for example for the production of tablets by wet or melt granulation granulation, direct compression or roller compaction process can be applied to the manufacturing of the immediate or extended release system.

In case of immediate release formulations the matrix tablets contain the functional components API, A, SSE which serve to achieve and maintain supersaturation and optionally one or more additives e.g. lubricants, anti-sticking agents, binders, preservatives, sweetening agents, meltable excipients used for melt extrusion, separating agents, plasticizers, pigments, fillers, diluents, chelating agents, excipients used for final coating and the like.

In case of extended release formulations the matrix tablets contain the functional components API, A, SSE which serve to achieve and maintain supersaturation, excipients I-ER and/or D-ER to achieve extended release properties and optionally one or more additives e.g. lubricants, anti-sticking agents, binders, preservatives, sweetening agents, meltable excipients used for melt extrusion, separating agents, plasticizers, pigments, fillers, diluents, chelating agents, excipients used for final coating and the like.

The skilled person is readily able to produce such tablets without undue burden.

It is also possible to have a bilayer tablet with one immediate release layer and one extended release layer of Flibanserin.

Another technique to obtain extended release tablets is to spray retarding lacquers onto immediate or moderately extended relase tablets. Thus an even more "tailored" release of the active ingredient, fitting exactly the need for flat plasma level profiles after oral intake can be obtained.

### Pellets

The formulations of the present invention embrace also pellet formulations. For example, the present invention provides for but is not limited to pellet formulations having the following exemplary composition for administration, particularly oral administration, of Flibanserin, comprising
A) a core material containing or consisting of one or more pharmaceutically acceptable pH modifiers;
B) optionally an insulating layer,
C) a layer containing or consisting of Flibanserin and the supersaturizing excipient;
D) in case of extended release formulations a layer, which preferably represents a controlled release layer, containing or consisting of one or more pharmaceutically acceptable polymers having anionic or no ionic groups, preferably with pH-dependent solubility

The core material contains at least one pH modifier as defined above. Since the pH modifier is spatially separated from Flibanserin in the formulation of the above described release system it remains stable when stored, undesirable interactions between pH modifier and Flibanserin are prevented. Only after the oral administration of the immediate or extended release system of the present invention the pH modifier does dissolve and produces a micro environment in which Flibanserin can dissolve.

The content of the pharmaceutically acceptable pH modifier(s) is usually between 80 and 100% in the core material.

The core material which may be spherical, has preferably an average diameter of 0.1-5 mm, more preferably 0.2-2 mm and most preferably 0.4-0.8 mm.
The core can be manufactured by techniques generally known in the art such as direct pressing, extrusion and followed by forming to preferably rounded shape, moist or dry granulation or direct pelleting, for example on plates or rotor pelletizers, or by binding of powders, such as powder layering on spherules (nonpareils). The core which is free of Flibanserin can be homogeneous or can have a layered structure or any other build-up known by those skilled in the art.

Furtheron, the core may optionally be coated with an insulating layer. To coat the core material before the application of the further layer(s) with an insulating /mobility decreasing layer based on a water-soluble, pharmaceutically acceptable polymer may be advantageous for two reasons:
I) To increase the durability of the finished core product material.
II) To decrease the mobility of the pH modifier and control interactions between the pH modifier and the active pharmaceutical ingredient
Most suitable are water-soluble polymers including gum arabic or a partially or totally synthetic polymer selected from the alkyl celluloses and derivatives thereof. The insulating layer can be manufactured by techniques generally known in the art.

Either on the core or in case of the use of a optional insulating layer on the this latter layer, a layer comprising Flibanserin and the supersaturizing agent(s) is applied according to procedures known to the skilled person.

The active substance layer contains Flibanserin (as described in API)) as well as preferably one or more supersaturizing excipients (as described in SSE)), if necessary also binders (as described in B)) and/or optionally one or more other excipients e.g. talc as glidant as described before.

The composition and application quantity of this layer is based on the most suitable relationship of Flibanserin to pH modifier and supersaturizing excipient.

Optionally an "onion" principle can be applied by spraying e.g. a layer of active substance and supersaturizing excipients (1/3 of total mass), than a layer containing acid(s) binder and glidant (1/2 of total mass), another layer of active substance and supersaturizing excipients (1/3 of total mass), another layer containing acid(s) binder and glidant (1/2 of total mass) and finally another layer of active substance and supersaturizing excipients (1/3 of total mass).

If the manufacture of a extended release formulation is intended, an extended release coating layer can be applied on the layer comprising flibanserin. This extended release layer comprises one or more pH dependent or pH independent polymers or a mixture thereof as retarding agents( I-ER and/or D-ER) and optionally an additional water soluble polymer which serves as pore former. This polymer is not limited according to the present invention. Any type of pharmaceutically acceptable polymer having anionic or no ionic groups may be used.

In a preferred embodiment of the controlled release system of the present invention the extended layer comprises a polymer selected from the group consisting of Eudragit^{®} S and Hypromellose-phthalate HP 55 combined with antisticking agent such as talc and pore formers such as triethylcitrate.

The release system of the present invention may be prepared according to conventionally known methods.

If starting material are Flibanserin pellets produced by extrusion, only the extended release coating applied according to known procedures is employed.

### Extrusion

The pellet layering described before offers the advantage that even excipients incompatible with the drug substance (e.g. acids) can be incorporated into the formulation as the layers are physically separated from each other during storage. As Flibanserin however is a very stable compound and is compatible with the excipients of the present invention, very effective dosage forms can also be obtained by more simple production modes. Therefore a simple production mode such as wet or melt extrusion is an even more preferred dosage form for preparation of pellets.
Formulations suitable for wet extrusion contain usually 25 - 30 % microcrystalline cellulose in order to achieve well extrudable mixtures which can be broken and shaped into round pellets by a spheronizer after the extrusion process. Disadvantage of this process is the reduction of achievable drug load due to the content of microcrystalline cellulose, which is non-efficaceous with regard to dissolution and supersaturation. It has been found quite unexpectedly that production of extrudates is feasible without adding microcrystalline cellulose when part of the supersaturizing agent is dissolved and used in solution as wetting agent for wet extrusion. Thus a formulation which contains 100 % of active ingredient and functional excipients with regard to dissolution and supersaturation is feasible, in some cases where sticky extrudates are produced, it has been found quite unexpectedly, that low amounts (about 1% w/w) of colloidal silicium dioxide (Aerosil) reduce stickiness quite effectively.
For melt extrusion about 20% of plasticizer is needed normally in order to get an extrudable mass. Disadvantage of this process is the reduction of achievable drug load due to the content of plasticizer, which is non-efficaceous with regard to dissolution and supersaturation. It has been found quite unexpectedly that production of extrudates is feasible without adding a standard plasticizer if thermoplastic or meltable acids or combination of solid and liquid acids are used or if a meltable excipient which achieves extended release properties is applied. Thus a formulation which contains 100 % of active ingredient and functional excipients with regard to dissolution, supersaturation and extended release properties is feasible. Furthermore it was even more unexpected,that special combinations of acids and supersaturizers results in synergistic effects with regard to dissolution at high pH values. Quite unexptectedly heating to about 120°C in the mid of the extrusion barrel results in further improvement of dissolution.

In the following the wet extrusion process of the present invention is described in more detail.
All solid components are weighed into a suitable vessel and then are thoroughly mixed in an adequate equipment e.g. a Turbula or cube mixer. If bulky material such as powdered tartaric acid is applied, the mixture is sieved, e.g. by a 800 µm sieve in order to get a homogenous mixture. Then this mixture is dosed with an appropriate feeder into an extruder. Preferred extruders are twin screw extruders, but other extruders like single screw extruders or screen type extruders are also suitable. The liquid, which may be either water or a solution of a supersaturizing excipient is dosed also into the extruder by appropriate pumps with exact dosing features (an alternative method would be to mix solids and liquid prior to extrusion, which is mandatory for screen type extruders) and then the material is extruded through dies of 0.5 to 3 mm, preferably are diameters from 0.6 to 1.4 mm, most preferred 0.6 - 0.8 mm. The extrudate is either cut at the front of the die by a face cut system or later on by either strang granulators or other suitable equipments. Then the material is shaped by a spheronizer to rounded balls which are then dried in an appropriate dryer. If necessary, the material is then sieved by appropriate sieves to remove too fine particles due to extrudates broken during spheronization or too large particles stemming from agglomeration of extrudates. Suitable particle sizes are 0.5 - 2 mm, more preferably 0.6 - 1.4 mm, most preferred 0.8 - 1.2 mm.
The final pellets can either be filled directly into capsules as immediate release formulations or matrix extended release pellets or further processed by spraying retarding lacquers onto the pellets or by production of tablets by combining pellets with appropriate excipients by tabletting processes. The resulting tablets can either be immediate release, extended matrix tablets if retarding excipients are used for tabletting and extrusion or extended release tablets formed by spraying retarding lacquers onto tablets.
If final goal are tablets, extrudates can also be milled after drying and then processed to tablets with appropriate excipients.

In the following the **melt extrusion** process of the present invention is described in more detail.
All solid components are weighed into a suitable vessel and then are thoroughly mixed in an adequate equipment e.g. a Turbula or cube mixer. If bulky material such as powderered tartaric acid is applied, the mixture is sieved, e.g. by a 800 µm sieve in order to get a homogenous mixture. Then this mixture is dosed with an appropriate feeder into an extruder which is heated to a temperature which achieves plastification of the total mass. This temperature is frequently about 3 to 10 °C below the melting point of the plasticizer, sometimes the most suitable temperature has to be determined experimentally especially if mixtures of plasticizers or mixtures of solid and liquid plasticizers are applied. Preferred extruders are twin screw extruders, but other extruders like single screw extruders are also suitable as long as temperature control is feasible. The material is extruded through dies of 0.5 to 3 mm, preferably are diameters from 0.6 to 1.4 mm, most preferred 0.6 - 0.8 mm. The extrudate is either cut at the front of the die by a face cut system or later on by either strang granulators or other suitable equipments. Then the material is shaped by a spheronizer to rounded balls at suitable temperatures which allow softening of the extrudate but still avoiding stickiness. Suitable temperatures are in many cases temperatures 3 - 10 °C lower than the extrusion temperature. With hygroscopic extrudates drying in an appropriate dryer may be necessary. If necessary, the material is then sieved by appropriate sieves to remove too fine particles due to extrudates broken during spheronization or too large particles stemming from agglomeration of extrudates. Suitable particle sizes are 0.5 - 2 mm, more preferably 0.6 - 1.4 mm, most preferred 0.8 - 1.2 mm.
Melt extrusion can also result directly in tablets if the die has slit with 3 - 6 mm thickness and a width fitting to a Calender system which is placed below the die. The Calender consists of two counterrotating cylinders with openings of the desired tablet size. Other processes which also result in direct tablet formation e.g. volumetric dosing systems are also feasible.

The final pellets can either be filled directly into capsules with immediate release formulations or matrix extended release pellets or further processed by spraying retarding lacquers onto the pellets or by production of tablets by combining pellets with appropriate excipients by tabletting processes. The resulting tablets can either be immediate release, extended matrix tablets if retarding excipients are used for tabletting and extrusion or extended release tablets formed by spraying retarding lacquers onto tablets.
If final goal are tablets, extrudates can also be milled after drying and then processed to tablets with appropriate excipients.

### Formulations obtained by spray drying

The most intimate mixtures of active ingredient and functional excipients are obtained by dissolving all materials in water or suitable organic solvents or mixtures thereof and subsequent spray drying, as this results in molecular dispersions in some cases amorphous active ingredient is obtained, which is better soluble than crystalline material.
As solubility of Flibanserin in water even together with acids is limited, organic solvents have to be used. Solubility of most supersaturizing excipients of the present invention in organic solvents however is very low Unexpectedly it was found out that if active ingredient and acid were dissolved in a mixture of tetrahydrofurane (THF), isopropanol and water in ratios 6:3:1, and Methocels were dissolved in isopropanol and water in ratios 9:1, and then mixed prior to spray drying, no precipitation occurred.
Spray drying can be done in appropriate dryers, e.g. a Buechi 290 spray dryer for smaller scales. Spray drying can be performed at inlet temperatures of 120 - 140 °C and spraying rates of about 200 g solution/hour.
The obtained spray dried powder can be used for further processing like wet or melt extrusion, pellet layering or tabletting employing roller compaction or wet granulation to improve the poor flowability of the spray dried powder etc as described in the previous sections.

### Packaging

If the extended release system which is subject of the present invention is a capsule, preferably it shall be of the capsule size of between 5 and 0. The capsule then comprises the pharmaceutical extended releases system in form of granules which correspond in their chemical and physical composition to the core of the tablet but which are smaller in size.

When core material with an average diameter of 0.4-1.5 mm is used, the process described above produces for example pellets containing Flibanserin, which can then be packed into capsules. To do this, a number of these units corresponding to the required dosage may be packed into capsules in a standard capsule filling machine. Suitable hard capsules include, for example, hard gelatine capsules or hard capsules of hydroxypropyl methylcellulose (HPMC). Alternatively these units may be compressed together with suitable binders into tablets which disintegrate in the stomach releasing the coated pellets.

The extended release system may be packed in bottles or blisters well known in the art. Among such blisters are such being made of polyvinylchloride or polyvinylidene chloride. Aluminum-blisters are also possible. Bottles may be made of poylpropylene or polyethylene for example. Optionally desiccants like silica gel or molecular sieves can be used in the bottles. Other conventional packaging materials are possible, too.

The immediate and extended release systems of the invention can be packaged in a container, accompanied by a package insert providing pertinent information such as, for example, dosage and administration information, contraindications, precautions, drug interactions and adverse reactions.

Accordingly the present invention also refers to a pharmaceutical package suitable for commercial sale comprising a container, an immediate or extended release system according to the present invention.

### BRIEF DESCRIPTION OF THE DRAWINGS

- Fig. 1: Fig 1 shows comparisons of dissolution curves obtained with reference examples and examples according to the present invention at pH 6.0;
- Fig. 2: Fig 2 shows dissolution profiles of formulation according to the invention (examples ExB01 - B17) compared to a reference (Ref05).
- Fig. 3: Fig 3 shows dissolution profiles of formulation according to the invention with high amounts of acids at pH 6.8.
- Fig. 4: Fig 4 shows dissolution profiles of coated extended release pellets according to the invention in comparison to the uncoated pellets at pH 6.0.
- Fig. 5: Fig 5 shows dissolution profiles of the coated extended release pellets according to the invention in comparison to the uncoated pellets at pH 6.8.
- Fig. 6: Fig 6 shows dissolution profiles of matrix extended release pellets according to the invention.
- Fig. 7: Fig 7 shows dissolution profiles of matrix extended release pellets compared to coated extended release pellets having identical amounts of tartaric acid and supersaturizer Methocel A4 according to the invention.
- Fig. 8: Fig 8 shows dissolution profiles of matrix immediate and extended release tablets produced either by direct extrusion with calendar technology (examples E or by pressing tablets with milled extrudates (examples F) according to the invention.
- Fig. 9: Fig 9 shows dissolution profiles of matrix immediate and extended release tablets produced by roller compaction according to the invention compared to reference products.

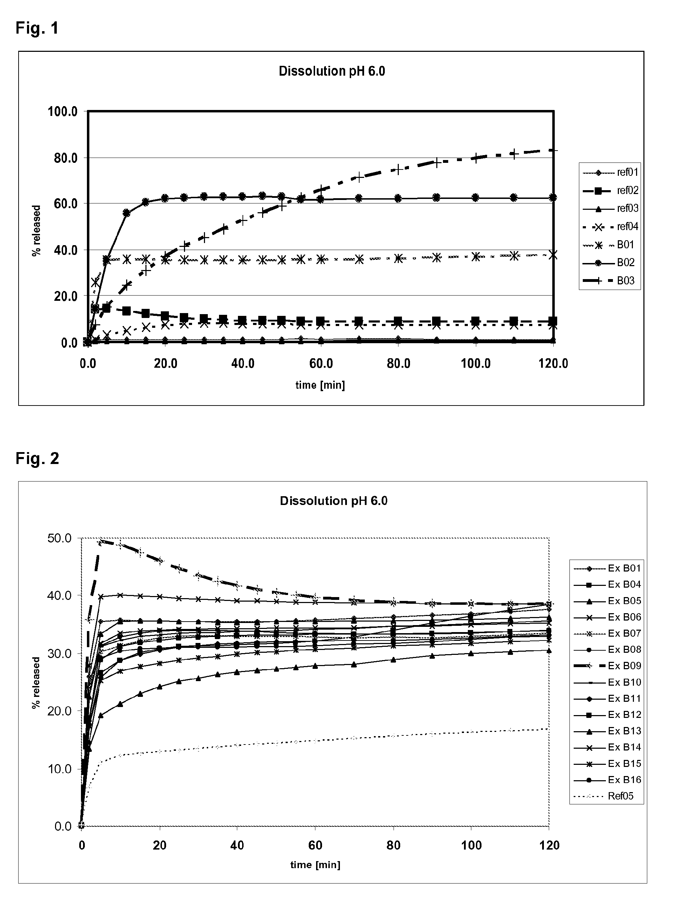

According to the present invention it is provided a virtually pH-independent release for the active substance Flibanserin which is a weak base and which in the range from pH 1 to pH 7.5 would exhibit pH-dependent solubility characteristics. That is Flibanserin has greater solubility under acidic conditions and less solubility under neutral and basic conditions. As a result the present invention provides a change of the release characteristics of Flibanserin resulting in a significantly improved bioavailability which is independent on the pH in the gastrointestinal tract when administered orally as shown in Fig 1.

The composition of the formulations described in Fig. 1 is given in Table Fig. 1a.

**Table Fig 1a: (all amounts indicate gram)**

| Batch | Ref 01 | Ref 02 | Ref 03 | Ref 04 | Ex B01 | Ex B02 | Ex B03 |
|---|---|---|---|---|---|---|---|
| Flibanserin | 100 | 100 | 100 | 100 | 100 | 100 | 100 |
| fumaric acid | | | | | | | 50 |
| tartaric acid | | 100 | | | 110 | 240 | 170 |
| lactic acid | | | | | 10 | | 20 |
| succinic acid Methocel F4M | | | | 94 | 20 | | 20 |
| Methocel A4C | | | 20 | 40 | | 40 | |
| Poloxamer 188 | | | | | | 30 | |
| microcryst. Cellulose | 100 | | | | | | |
| total | 200.0 | 200.0 | 120.0 | 234.0 | 240.0 | 410.0 | 360.0 |
| % acid | 0.0 | 50.0 | 0.0 | 40.2 | 50.0 | 58.5 | 66.7 |
| % supersaturizer | 0.0 | 0.0 | 16.7 | 17.1 | 8.3 | 9.8 | 5.6 |

**Table Fig 1b:**

| | | | | | | | |
|---|---|---|---|---|---|---|---|
| batch | ref01 | ref02 | ref03 | ref04 | B01 | B02 | B03 |
| % max | 1.3 | 14.4 | 0.7 | 8.1 | 37.6 | 62.9 | 82.7 |
| % AURC | 1.3 | 9.8 | 0.5 | 7.1 | 35.6 | 59.5 | 59.3 |
| % AURC_L/E | 1.2 | 0.8 | 1.6 | 1.4 | 1.1 | 1.2 | 2.4 |

| | | | | | | | |
|---|---|---|---|---|---|---|---|
| % max represents the highest release during dissolution testing % AURC represents the ratio of area under the release curve during dissolution testing compared to AURC for 100 % release % AURC_L/E represents the ratio of areas under the release curve during late and early time spans of dissolution testing, in this case time spans of 60 - 120 minutes was used for %AURC_L and 0 - 60 minutes was used for %AURC_E. Detailed explanations are given in section All Dissolution. | | | | | | | |

Figure 1 and Table Fig1b, which shows the dissolution parameters of the formulations given in Table Fig1a, show, that formulations ref 01 & ref 03, which contain no acid show nearly no release at all. Ref 02 which contains 50% of tartaric acid shows a maximal release of 14.4 % which however decreases to 9 % as no supersaturizers are present. Ref 04 contains 40 % succinic acid and a sufficient amount of supersaturizer, but due to the suboptimal amount and kind of acid, dissolution is only about 8%. Ex B01 contains the same percentage of acid as ref02, but also a suitable supersaturizer. This achieves an about threefold increase in % max which is maintained over the duration of dissolution testing as evident from a fourfold increase in %AURC.
Ex B02 comprises 58.5 % of tartaric acid and contains an adequate supersaturizer. This achieves a 4.4-fold increase in % max which is maintained over the duration of dissolution testing as evident from a six fold increase in %AURC. Compared to ExB01 a 1.7-fold increase in % max which is maintained over the duration of dissolution testing as evident from the same 1.7-fold increase in % AURC is achieved.

Ex B03 shows a formulation comprising a combination of acids and contains an adequate supersaturizer. This achieves a 5.7-fold increase in % max which is reached at the end of dissolution testing, a six fold increase in %AURC.
Compared to ExB01 a 2.2-fold increase in % max and a 1.7-fold increase in % AURC is achieved. Most unexpected however is the fact that the optimized amounts and combination of acids is not only superior with regard to %max but achieves an extended release effect: ExB02 is already close to %max at 10 minutes, ExB03 releases only about 25 % at 10 minutes and dissolution increases continuously. As ExB03 contains the same supersaturizer as ExB01, which shows even faster dissolution than ExB02, extended release seems to be caused by the acids, not by the supersaturizer.

Fig 2 shows dissolution profiles of formulations according to the invention (examples Ex B01 - Ex B16 of Table B1) compared to a reference, which contains hypromellosephthalate 55 as "supersaturizer" (Ref05). All formulations contain 10 parts of flibanserin, 11 parts tartaric acid, 1 part lactic acid and 2 parts of supersaturizer.
Most relevant dissolution parameters are given in Table Fig 2:

**Table Fig 2:**

| batch | Ex B01 | Ex B04 | Ex B05 | Ex B06 | Ex B07 | Ex B08 | Ex B09 | Ex B10 |
|---|---|---|---|---|---|---|---|---|
| %max | 37.6 | 38.5 | 36.2 | 40.0 | 33.5 | 34.0 | 49.4 | 35.6 |
| % AURC | 35.6 | 32.4 | 35.0 | 38.4 | 32.3 | 33.0 | 40.8 | 33.6 |
| | | | | | | | | |

| batch | Ex B11 | Ex B12 | Ex B13 | Ex B14 | Ex B15 | Ex B16 | Ref05 | |
|---|---|---|---|---|---|---|---|---|
| %max | 33.3 | 33.9 | 30.5 | 35.2 | 32.2 | 33.0 | 16.8 | |
| % AURC | 31.0 | 32.4 | 26.7 | 33.7 | 29.7 | 31.0 | 14.4 | |

It is evident that all Methocels used in the formulations Ex B01 - Ex B16 in figure 2 are suitable and markedly better than ref05, which is hypromellose phthalate 55. Among the Methocels Methocel A4M and Methocel E3 are slightly better than the other Methocels under these conditions.

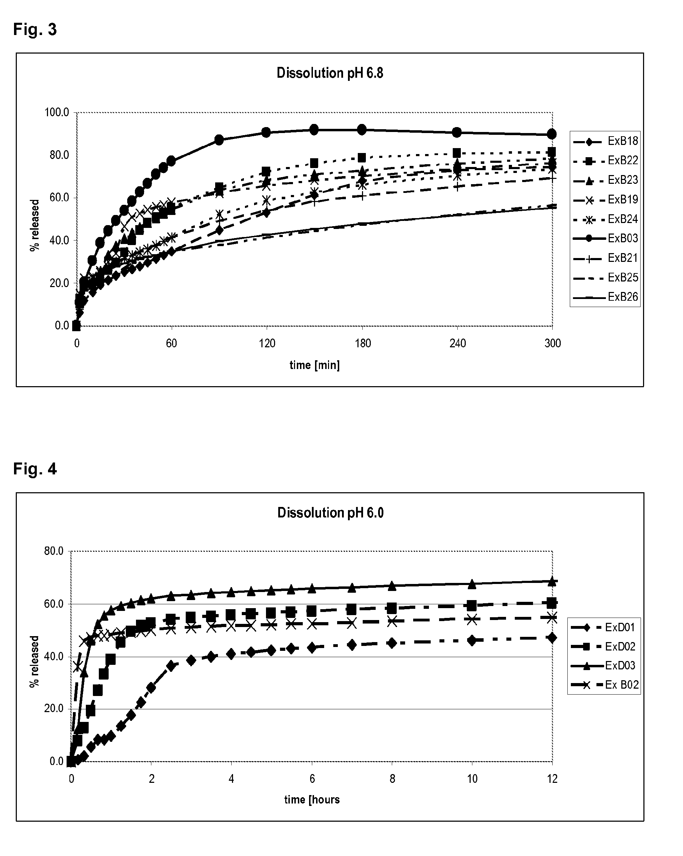

In Fig 3 dissolution profiles of formulations according to the invention with high amounts of acids at pH 6.8 are shown. All formulations (see Table B1) contain 10 parts of flibanserin, 5 parts fumaric acid, 17 parts tartaric acid, 2 parts lactic acid and 2 parts of supersaturizer.
Most relevant dissolution parameters are given in Table Fig 3:

**Table Fig 3**

| batch | ExB18 | ExB22 | ExB23 | ExB19 | ExB24 | ExB03 | ExB21 | ExB25 | ExB26 |
|---|---|---|---|---|---|---|---|---|---|
| %max | 74.2 | 81.5 | 78.5 | 76.0 | 73.5 | 91.9 | 69.1 | 56.4 | 55.2 |
| % AURC | 54.5 | 67.1 | 64.4 | 63.3 | 56.7 | 81.8 | 53.2 | 42.4 | 42.9 |

It is evident that all formulations shown in this table show adequate dissolution even at this high pH. They dissolved remarkably well if the extremely poor solubility of the active ingredient is considered.

Fig 4 shows dissolution profiles of coated extended release pellets according to the invention in comparison to the uncoated pellets at pH 6.0. All coated pellets contain an extrusion pellet according to ExB02 but different retarding lacquers, given in Table D. Release of all coated pellets is slower than of uncoated pellets. As expected, release of ExD03 which contains a high amount of HPMCP55 and thus dissolves at pH 6.0 is faster than with ExD02 despite the thicker coating layer. ExD02 is faster than ExD01 which corresponds to the thickness of the retarding lacquer.

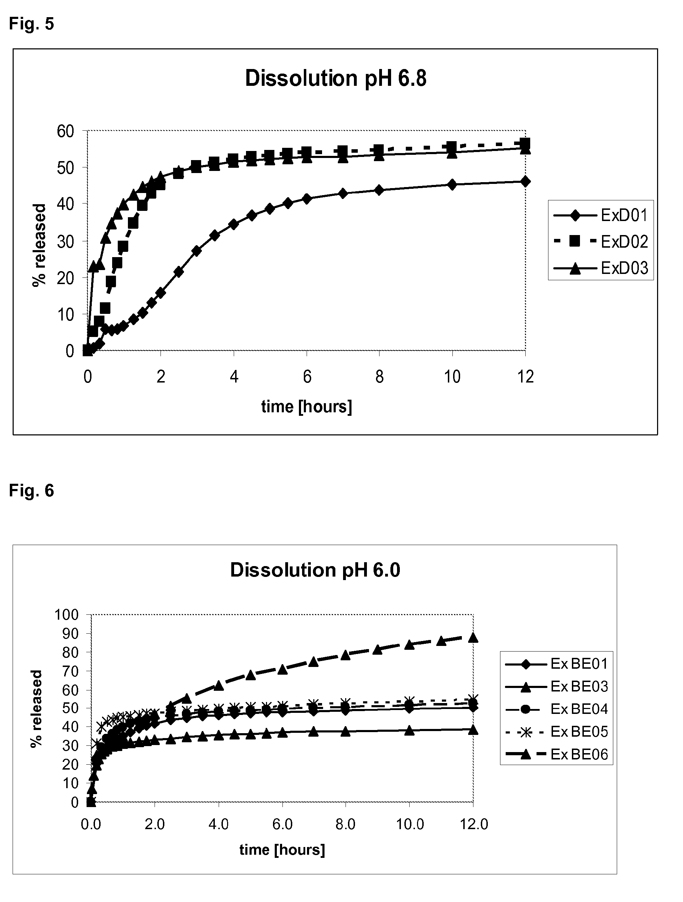

Fig 5 shows dissolution profiles of the the same coated extended release pellets as already described in Fig. 4 but this time at a pH 6.8. given in Table D. It is evident that even at this high pH where solubility of the active ingredient is extremely low, adequate dissolution occurs.

Fig 6 shows dissolution profiles of matrix extended release pellets (see Table B2) according to the invention.,Ex BE06 shows the best extension of release as well as the highest final release. This shows again that the combination of the three pH modifiers is also very suitable for extended release formulations.

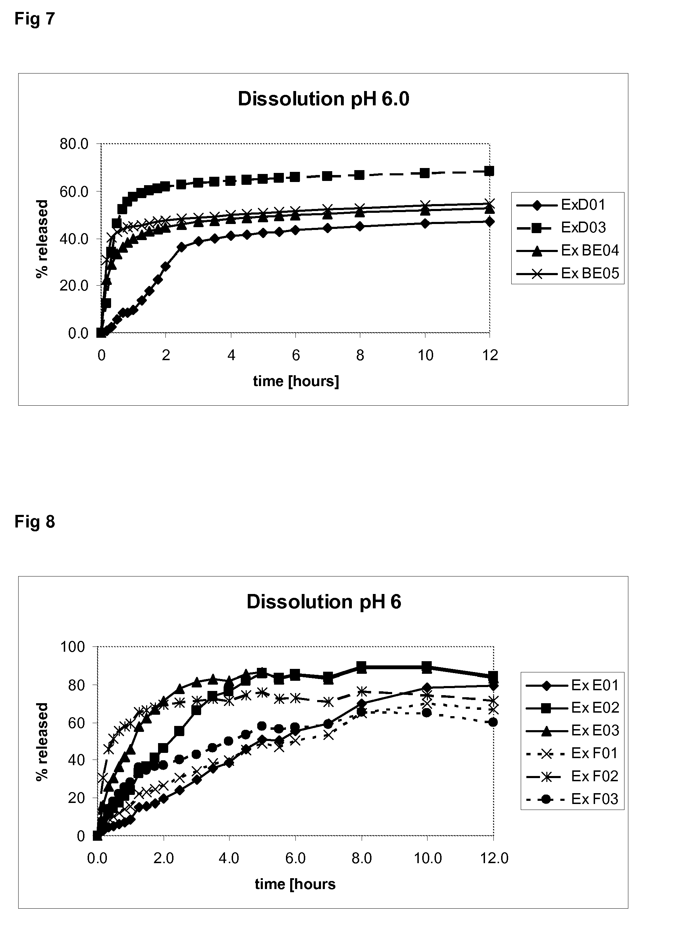

Fig 7 shows dissolution profiles of matrix extended release pellets (compared to coated extended release pellets (see Table D, Ex D1 - Ex D3) having identical amounts of tartaric acid and supersaturizer Methocel A4 according to the invention. Ex BE04 corresponds to Ex D01, (high amount of Eudragit S low amount of HPMCP55) . Ex BE05 corresponds to Ex D03 (low amount of Eudragit S high amount of HPMCP55), It is evident that in both cases the formulation containing a higher amount of Eudragit S shows the expected slower release than with higher amount of HPMCP 55, but also that the type of formulation has a certain influence on the release pattern.

Fig 8 shows dissolution profiles of matrix immediate and extended release tablets produced either by direct extrusion with calender technology (examples Ex E01 - Ex E03) or by pressing tablets with milled extrudates (see Table F) according to the invention. Ex E01 - Ex E03 are identical in composition which is according to Ex B03, tablet thickness with Ex E01 was 5 mm, thickness with Ex E02 was 3 mm and thickness with Ex E03 was 1.5 mm, It is evident that reduction of diameter in examples E results in increased dissolution rate with similar and high final dissolution. By inclusion of disintegrants (example ExF02) a faster dissolution is obtained than with a 3-layer tablet having disintegrant between two active ingredient layers (example ExF02). ExF01 is slower than tablets with disintegrants. Final release of examples E is slightly higher than with examples F.

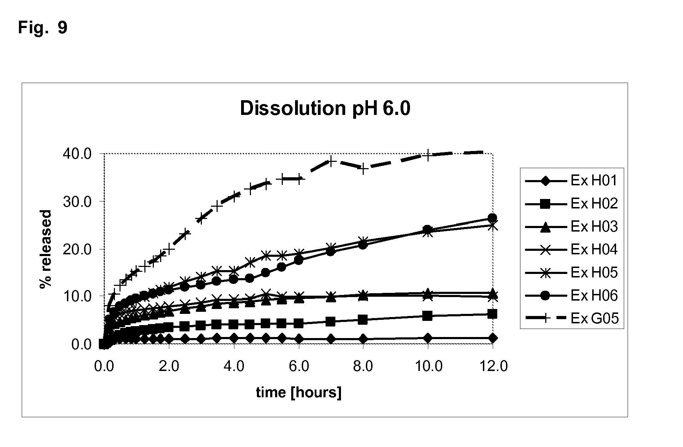

Fig 9 shows dissolution profiles of matrix immediate and extended release tablets produced by roller compaction according to the invention (Ex H03 - Ex H06) compared to reference products (Ex H01 - Ex H02). It is evident that relative ranking between Ex H03 and Ex H06 and compared to the reference formulations is similar to extrudates, dissolution of all batches however is somewhat lower than with extrudates, as evident from the comparison to Ex G05.

### EXAMPLES

### Al) Selection of most suitable acids and supersaturizing excipients

The process to identify the classes of most suitable supersaturizers and within classes the most suitable excipients is done as follows.

Preparing the formulations is done by mixing predissolved active ingredient and pH modifier and solutions of the excipient achieving supersaturation. Maintenance of the supersaturation behaviour is tested by adding buffer at the pH of minimum solubility of the active substance and measuring absorption by UV using an automated minidissolution device each 5 minutes for 30 minutes. Thus a particularly high sample throughput can be achieved.

For determining the optimum composition, the formulations were formed by pipetting together pre-prepared solutions into small vessels. As solubility of flibanserin in water is too low even at acidic conditions, 1g of flibanserin and 0.25 g tartaric acid were dissolved in a mixture of 30 ml tetrahydrofurane, 15 ml of isopropanole and 5 ml water, resulting in a concentration of 20 mg/ml for flibanserin and 5 mg/ml tartaric acid. Excipients were dissolved in water at a concentration of 10 mg/ml.
500 µl of solution containing flibanserin and tartaric acid and 400 µl solution containing potential supersaturizer (in case of the reference standard, water without supersaturizer was added) were mixed intensively and then transferred directly into the release vessel, which contained 19.1 ml of buffer conditioned at 37°C. Precipitation was determined in a miniaturized apparatus similar to conventional paddle dissolution test systems using standard dissolution software. Measurements were performed each 5 minutes from 3 to 28 minutes.

It is evident, that supersaturation is quite different for different excipients. As comparison of a large number of curves is difficult, a special mode of evaluation was developed: Area under curve is calculated over the time span of investigation and given as percentage of area for 100 % dissolution. This percentage reflects the amount of active ingredient which is available for absorption. By ranking of all results in ascending order, the most suitable excipients can be identified. Results of formulations of examples A1 - A23 are given in Table A.

**Table A: Percentage of % AURC in ranked order of Examples A1 - A23**

| Example | Supersaturizing excipient | % AURC |
|---|---|---|
| ex A04 | Kollidon K25 | 26.7 |
| ex A21 | Natrosol HX | 26.9 |
| ex A01 | none | 27.2 |
| ex A02 | PEG6000 | 27.4 |
| ex A03 | Plur F68 | 27.5 |
| ex A23 | Tween 80 | 29.3 |
| ex A22 | Hydroxypropylcellulose | 29.4 |
| ex A20 | Carbopol 971 | 30.9 |
| ex A05 | Koll idon va64 | 32.3 |
| ex A11 | **Methocel K15 MCR** | 44.2 |
| ex A08 | **Meth K100 MCR** | 44.7 |
| ex A15 | **Methocel E3** | 46.4 |
| ex A12 | **Methocel F4M** | 46.8 |
| ex A14 | **Methocel E4MCR** | 46.9 |
| ex A13 | **Methocel E15LV** | 46.9 |
| ex A17 | **Methocel E6** | 47.0 |
| ex A16 | **Methocel E5** | 48.3 |
| ex A06 | **Methocel A15LV** | 49.2 |
| ex A19 | **Methocel K100LV** | 49.8 |
| ex A07 | **Methocel A4C** | 51.4 |
| ex A10 | **Methocel F50** | 52.7 |
| ex A09 | **Methocel A4M EP** | 53.6 |
| ex A18 | **Methocel A15C** | 54.1 |

Table A shows that several excipients, which show good supersaturation with other active ingredients were completely ineffective with Flibanserin, as there was no apparent difference to the reference sample A01, whereas Methocels were quite effective. If 50 % improvement is used as marker, 14 excipients showed excellent supersaturation and 4 excipients showed nearly twice the AUC of the reference. It is evident that all Methocel types representing methylcelloluse, hypromellose 2208, hypromellose 2906 and hypromellose 2910 were effective. Therefore, these classes represent suitable supersaturizers and were used for development of optimised solid formulations. As dissolution from solid formulations depends on many different factors, relative ranking of Methocels from solid formulations is not necessarily identical to the ranking obtained with the dissolution test, all Methocel types were investigated during optimisation of solid formulations.

### All) Dissolution

The formulations of the invention are characterized by special dissolution testing predictive for absorption in man, which is not possible with conventional dissolution tests:
Conventional dissolution testing employs unphysiologically high volumes of 500 or 900 ml and are performed under sink conditions, that means the active principle is completely soluble. For a drug poorly soluble at pH >3 like Flibanserin the in vivo situation may be completely different in case of a non-acidic stomach and for the GIT where absorption occurs, these test conditions never reflect the real situation.
The dissolution testing employed for the formulations of the invention is much closer to the situation of oral drug intake:
- A volume of 200 ml is used which reflects the amount of water taken together with the medication e.g. in phase I studies
- pH of the buffer system is adjusted to a value where solubility of the active principle is minimal
If high dissolution is achieved under these conditions, which reflects a worst case scenario of the in vivo situation, dissolution and absorption will occur also in patients after oral drug intake. As expected dose of Flibanserin is 50 - 100 mg, a concentration of 0.5 mg/ml is used.

The impact of excipients according to the invention on in vitro dissolution at pH >= 5 which are important for absorption after oral intake is given below. Dissolution was done in a apparatus USP II (paddle method) at 60 rpm or in a miniaturized paddle apparatus with similar characteristics. Buffers were 0.05 M phosphate buffer at pH 5.0, 5.5, 6.0 and 0.02 M at pH 6.8. Determination of release was done by UV-Measurement at 278 nm using Agilent® software.

As it is difficult to compare large numbers of dissolution profiles, comparison was done by condensing the information to parameters such as maximal release (% max) and calculation of area und the release curve (AURC). If area is given as percentage of AURC versus AURC of 100 % release over the dissolution time, a figure (% AURC) which is independent from time span of dissolution evaluation is obtained. % AURC represents the average dissolution during dissolution testing.

For immediate release formulations % AURC is the most reliable parameter for ranking of formulations.

For extended release formulations % AURC is less reliable for ranking of formulations, as a slow release which decreases % AURC is an advantage. Therefore % AURC is not only calculated over the complete time span of dissolution testing, but also for "early" and "late" time spans. A high ratio of late over early % AURC (% AURC_L/E) together with an acceptable total % AURC indicates an adequate extended release formulation. As suitable time spans depend on release characteristics, details will be given for the individual figures.

### Example B: immediate release pellets and matrix extended release pellets produced by melt extrusion filled into capsules

The preparation of the immediate or extended release melt extrusion pellets of the present invention in the following Example usually takes place over 5 steps:
step 1): milling, sieving of excipients
step 2): preparation of the mixture
step 3): melt extrusion
step 4): cutting and rounding to spherical pellets
step 5): filling into capsules

The equipment described below can be replaced by other pieces which fulfill the same or similar features.

### step 1): milling, sieving of some excipients

Excipients with a particle size > 500 µm, e.g. poloxamer 188 are milled with a Retsch mill ZM 200, sieve size 500 µm at 6000 rpm and then sieved through a sieve with pore size 500 µm before manufacture of extrudates. If bulky material such as powderered tartaric acid is applied, it is sieved, through a sieve with pore size 800 µm.

### step 2): preparation of the mixture

All components of the melt extrudate (flibanserin, one or more acid(s) [if lactic acid is used, it is dropped into the powder mixture], one or more supersaturizing excipients, optionally a plasticizer and/or an extended release polymer) are weighed (amounts are given in table B1 as g per batch) into a suitable vessel and then are thoroughly mixed in a Turbula T2C mixer. Then the mixture is sieved through a 800 µm sieve in order to get a homogenous mixture.

### step 3): melt extrusion

The mixture obtained from step 2 is dosed with a Threetec ZD 12 F twin screw feeder into a 16 mm Haake Polylab TW 16 twin screw extruder with L:D (length of barrel over diameter of screws) ratio 24 : 1, temperature of the barrel is adjusted to 75 °C, which achieves plastification of the total mass and thus is suitable for extrusion. The material is extruded with a rotation speed of the screws of 250 rpm through a die with two horizontal openings of 1 mm diameter. The extrudates strangs are cooled on a running belt positioned directly below the die in order to avoid sticking. At he end of the running belt the extrudates are at room temperature and are solid and not sticking any more.

### step 4): cutting and rounding to spherical pellets

The extrudate is cut by a Thermomix cutter of Vorwerk company in fractions of 250 g. Speed setting is 7, cutting time is 10 seconds. Then the material is shaped by a Gabler spheronizer, diameter 250 mm to rounded balls at 75 °C and a rotation speed of 500 rpm. The material is then sieved by through sieves of 0.8 and 1.25 mm, the fraction with size between 0.8 and 1.25 mm is used for filling into capsules.

According to the aforementioned processes the following pellets may be prepared (amounts are given in table B1 or B2 as g per batch):

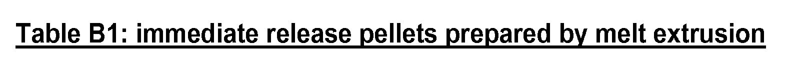

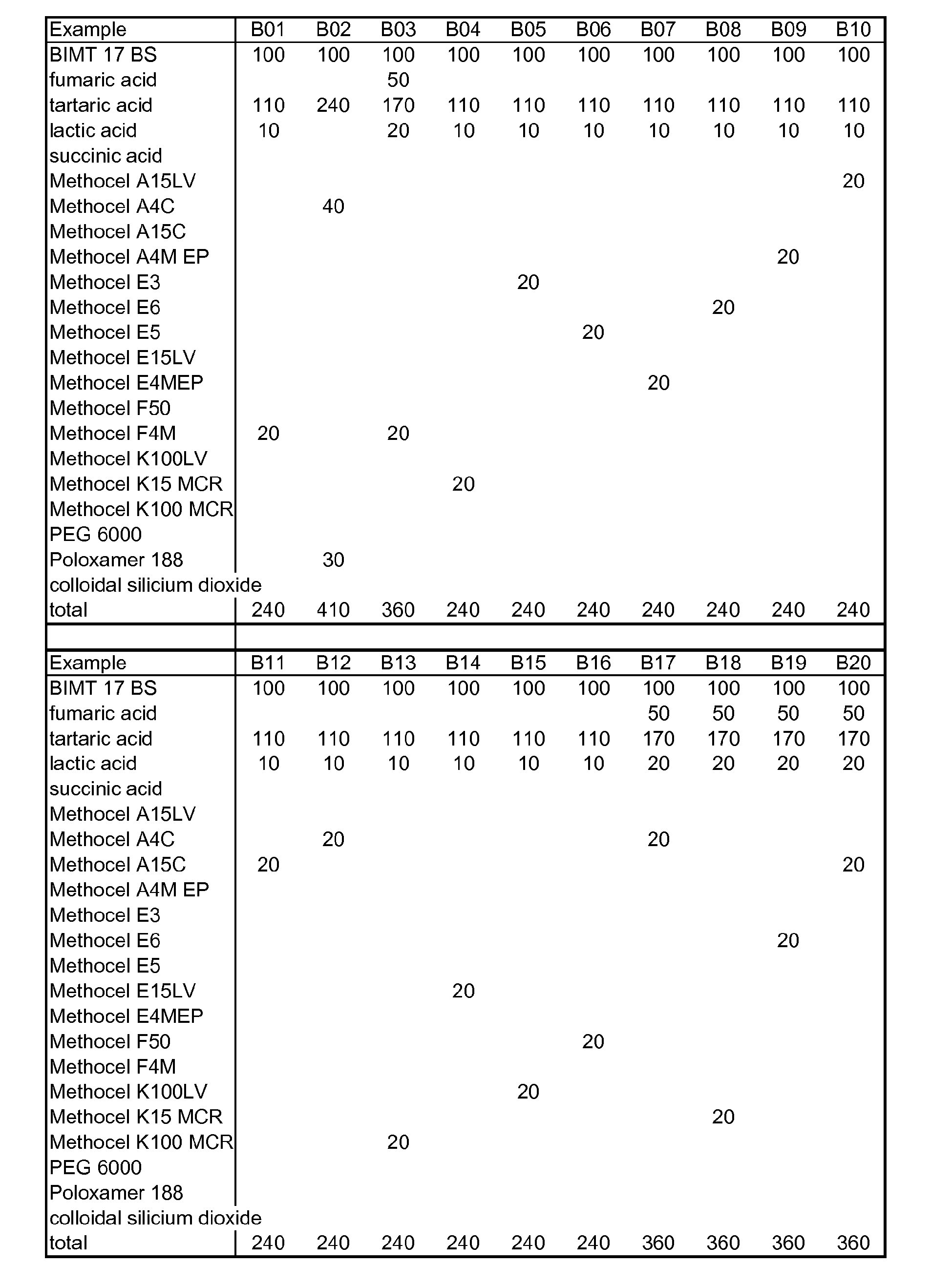

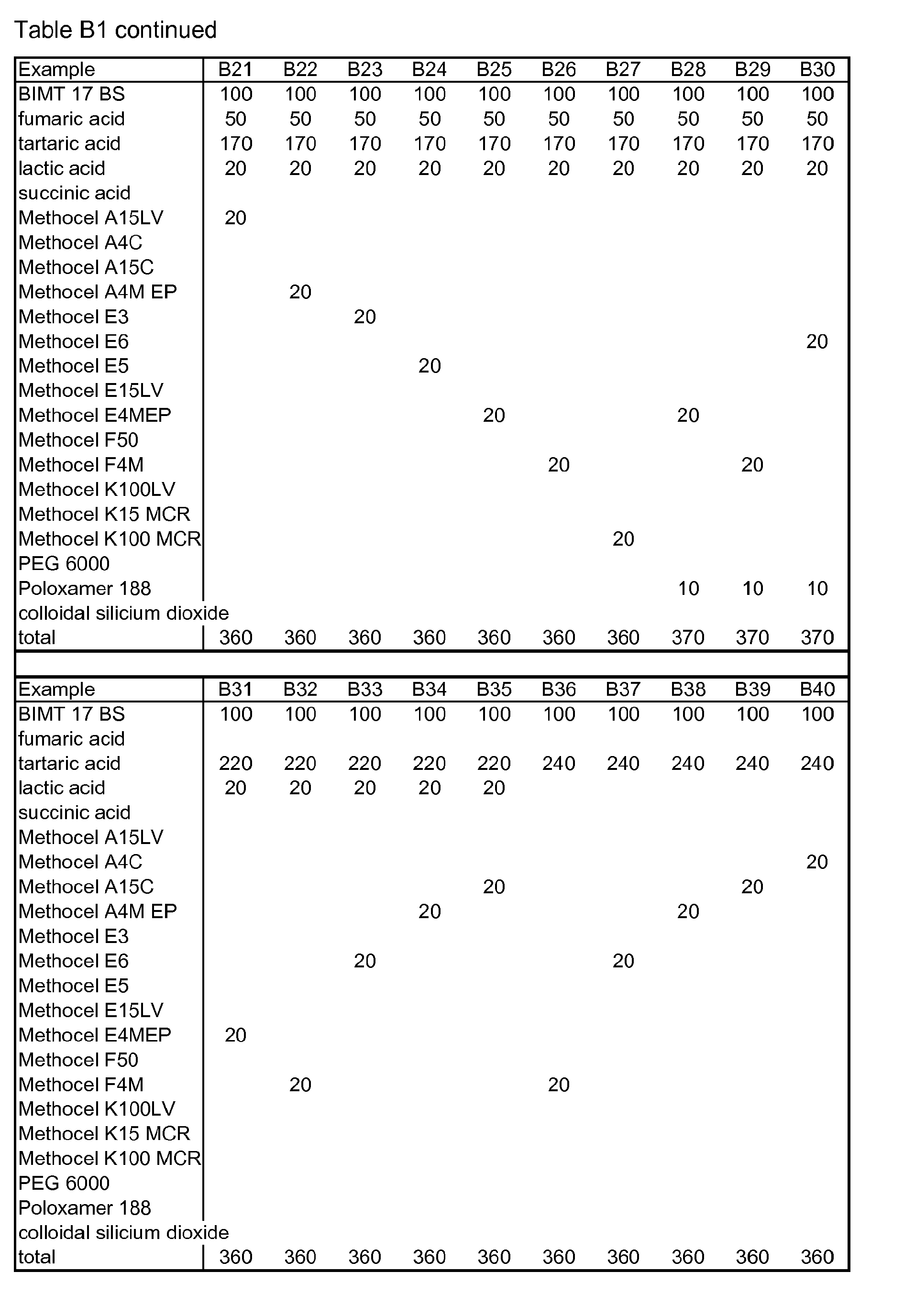

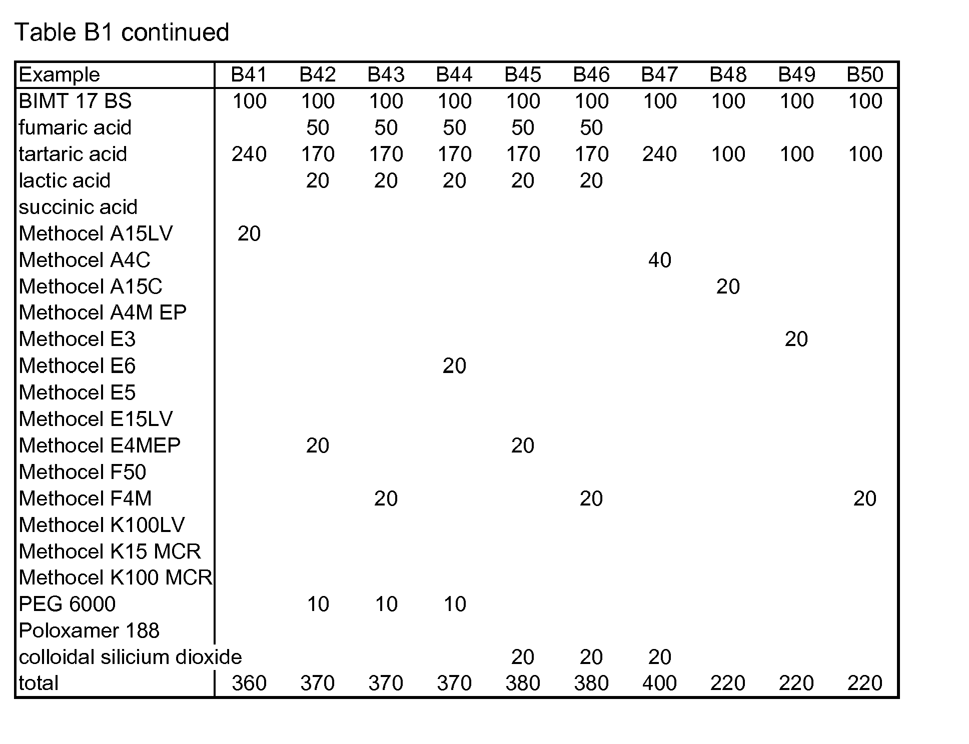

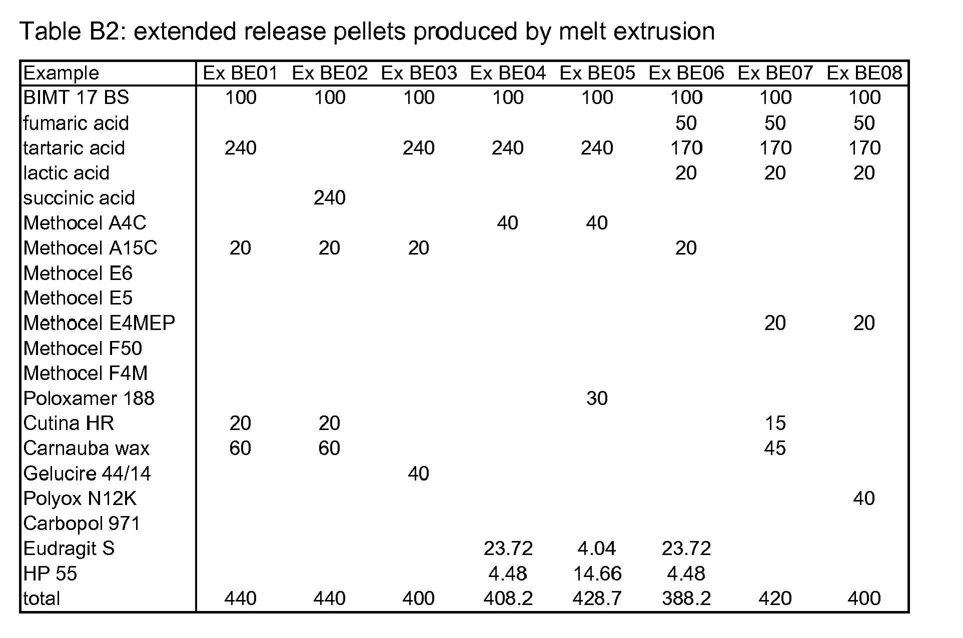

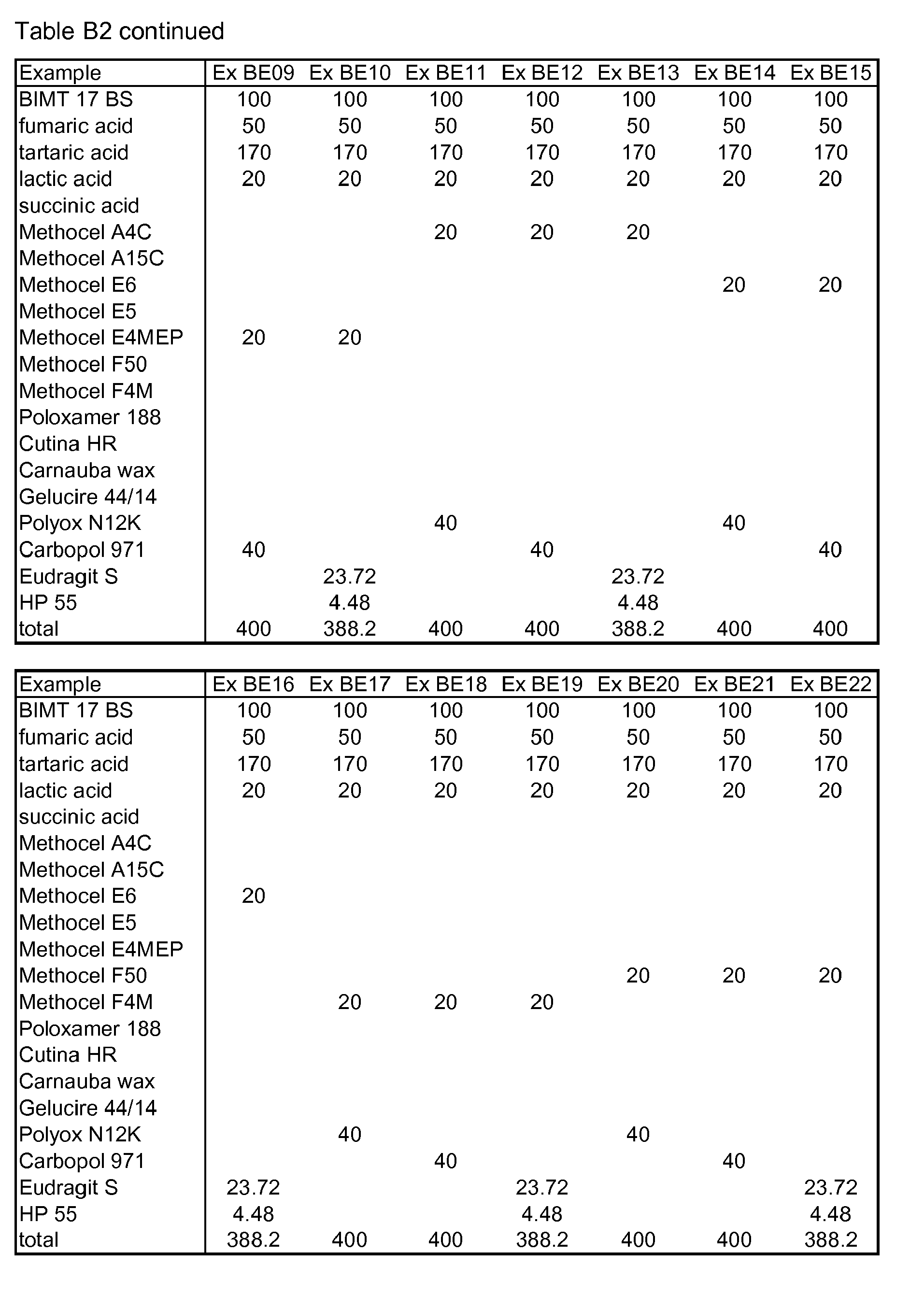

### step 5): filling into capsules

To the pellets obtained from step 4, talc is added in amount of 0.5 % of pellet weight (e.g. if 200 g pellets are used amount of talc is 1 g) and then mixing is done for 5 minutes in a Turbula T2C mixer. Then capsules are filled automatically into HPMC or hard gelatine capsules size 0 using an In-cap capsule filler. Incorrectly filled capsules are sorted out by a Mocon AB-Plus sorter.

### Example C: immediate release pellets and matrix extended release pellets produced by wet extrusion filled into capsules

The preparation of the immediate or extended release melt extrusion pellets of the present invention in the following Example usually takes place over 4 steps:
step 1): preparation of the mixture
step 2): wet extrusion
step 3): cutting and rounding to spherical pellets
step 4): filling into capsules

The equipment described below can be replaced by other pieces which fulfill the same or similar features.

### step 1): preparation of the mixture

All components of the melt extrudate (flibanserin, one or more acid(s) [if lactic acid is used, it is dropped into the powder mixture], one or more supersaturizing excipients, optionally a plasticizer and/or an extended release polymer) are weighed (amounts are given in table B1 or B2 as g per batch) into the vessel of a Thermomix Mixer and then are thoroughly mixed for 30 seconds with speed 5. Then an appropriate amount of water suitable for wet extrusion is added to the mixture and then again mixed for 60 seconds with speed 7. As some components dissolve slowly which results in a change of viscosity, mixing is repeated after 15 minutes storage.

### step 2): wet extrusion

The mixture obtained from step 1 is dosed with a Threetec ZD 12 F twin screw feeder into a 16 mm Haake Polylab TW 16 twin screw extruder with L:D ratio 24 : 1, temperature of the barrel is roome temperature. The material is extruded with a rotation speed of the screws of 120 rpm through a die with two horizontal openings of 1 mm diameter. The extrudates strangs fall on a running belt which is positioned directly below the die. In order to avoid sticking, air warmed to about 50 °C is blown onto the belt. At he end of the running belt the extrudates are dried at the surface and not sticking any more.

### step 3): drying, cutting and rounding to spherical pellets

The extrudates are dried at 50 °C for 12 hours. Then the extrudate is cut by a Thermomix cutter of Vorwerk company in fractions of 200 g. Speed setting is 7, cutting time is 10 seconds. Then the material is shaped by a Gabler spheronizer diameter 250 mm to rounded balls at 75 °C and a rotation speed of 500 rpm. The material is then sieved by through sieves of 0.8 and 1.25 mm, the fraction with size between 0.8 and 1.25 mm is used for filling into capsules.

According to the aforementioned processes pellets according to the compositions given in Tables B1 & B2 but optionally with an additional amount 1.5 % w/w colloidal silicium dioxide of total mass may be prepared

### Example D: extended release pellets produced by melt or wet extrusion and subsequent spraying of retarding lacquers onto the pellets, filled into capsules

The preparation of the immediate or extended release melt extrusion pellets of the present invention in the following Example usually takes place over 6 steps:
step 1): preparation of the mixture;
step 2): preparation of the binding solution
step 3): melt extrusion
step 4): cutting and rounding to spherical pellets
step 5): spraying of extended release coating onto the pellets.
step 6): filling into capsules

### Steps 1) to 4) and step 6) are identical to procedures 1) to 4) and 5) described in Example B, only step 5) is applied additionally

### step 5): spraying of extended release coating onto the pellets

### 5a) Preparation of the Lake Solution for pH-dependent polymers

Isopropyl alcohol and acetone were charged in a suitable reaction vessel then Eudragit S100 and hypromellosephathalat 55 were added in portions and dispersed in this solution with stirring, till complete dissolution occurred. Then water and triethyl citrate were added slowly in order to maintain a clear solution. Then talc was added under stirring. The amounts of solvents and excipients are given as gram in Table D. The solution was stirred continously also during spraying

### 5b) Preparation of the Lake Solution for pH-independent polymers

Isopropyl alcohol and water were charged in a suitable reaction vessel then Ethylcellulose and PEG 6000 were added in portions and dispersed in this solution with stirring, till complete dissolution occurred. Then talc was added under stirring. The amounts of solvents and excipients are given as g in Table D. The solution was stirred continously also during spraying

### 5c) Spraying of the obtained lake solution

The lake solution obtained from step 5a) or 5b) was sprayed onto the melt extrusion pellets (amount of pellets is given as g in Table D. To this purpose the pellets were placed in a suitable Huettlin Microlab fitted with an air inlet and exhaust. At an air inlet temperature of about 45°C the product was sprayed with the lake solution in continuous operation and sprinkled so as to produce roughly spherical particles. The following conditions were used:

| | |
|---|---|
| inlet air quantity | 12 m³/h preferably |
| spraying rate | 0.3 - 1.5 g/min (rate is increased gradually during spraying) |
| spray pressure | 0.6 bar, |
| micro climate | 0.3 bar |
| nozzle diameter | 0,8 mm |
| spray time | about 1.5 h |
| product temperature | 30 - 35°C |

The virtually spherical product obtained was then dried in a suitable drying device at 40°C for 12 hours. The product was fractionated using a suitable screening machine with perforated plates having nominal mesh sizes of 0.8 and 1.25 mm, the fraction between 0.8 and 1.25 mm was used for capsule filling.

According to the aforementioned processes the following pellets may be prepared, using starterpellets produced by either melt extrusion or wet extrusion according to the examples described in Table B1:
Table D Composition of retarding lacquers (starterpellets are used in amount containing 100 g active ingredient numbers in the table indicate gram.

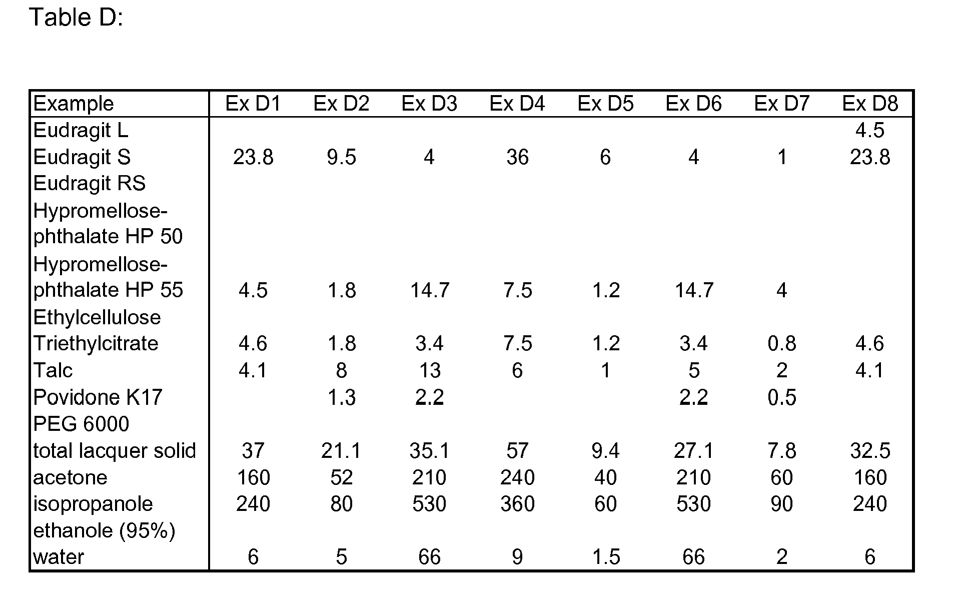

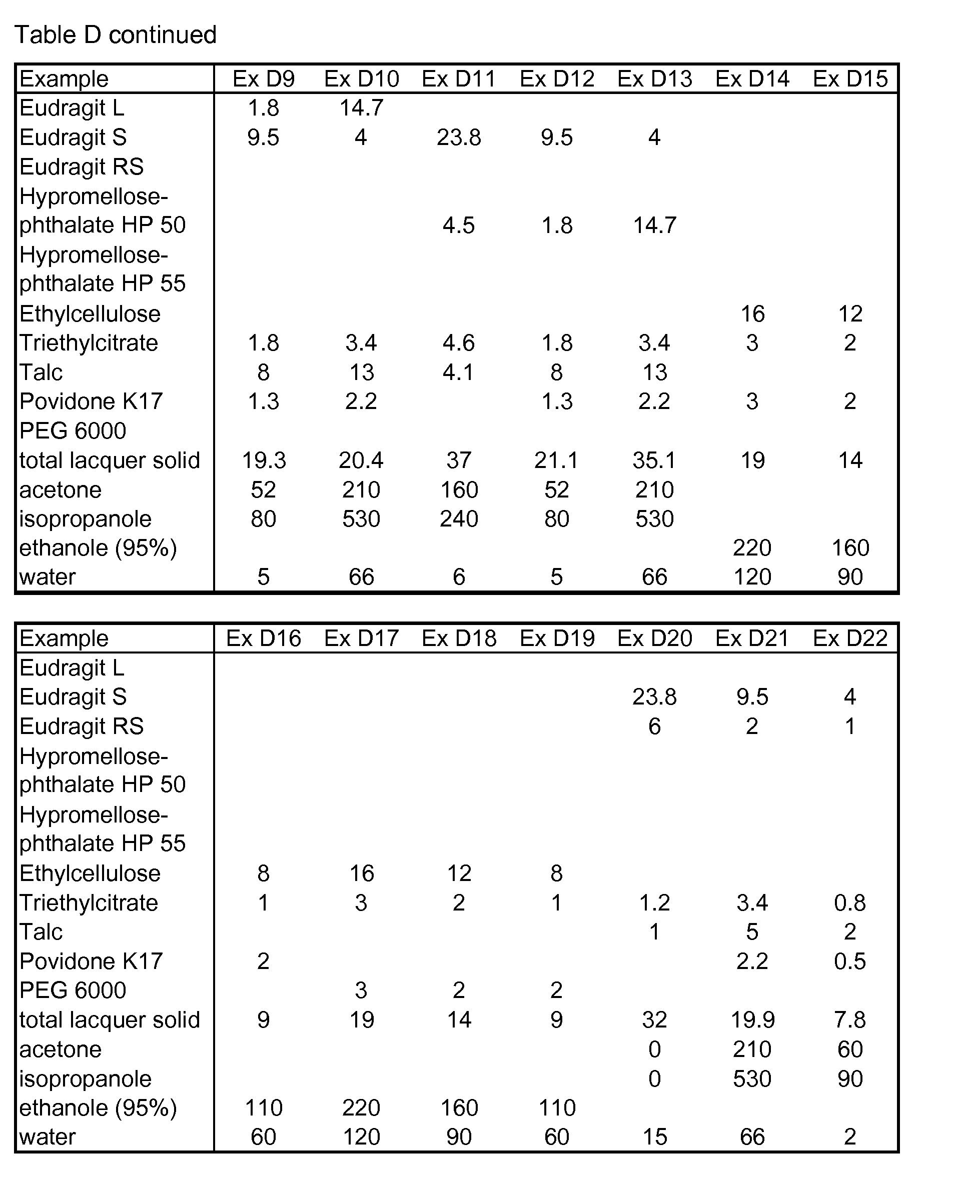

### Example E: extended release matrix tablets produced by melt or wet extrusion

The preparation of the immediate or extended release matrix tablets of the present invention in the following Example usually takes place over the following steps:
step 1): milling, sieving of excipients (only melt extrusion)
step 2): preparation of the mixture
step 3): wet or melt extrusion using a die with horizontal slit
step 4): formation of tablets by a calendar sytem
step 5):"smoothing" of tablets
step 6):packing into bottles or blisters

The equipment described below can be replaced by other pieces which fulfill the same or similar features.

Steps 1)- 3) are identical to the steps described in Examples B & C, the only difference is that instead of a die with holes (which produces round strangs with diameter according to the die hole) a die with horizontal slit is used. This produces a flat extrudate of even width and thickness, which is fed directly into a calender which is placed below the die.

Step 4)
The calender consists of two counterrotating cylinders of equal size, containing rows of openings which are half of the produced tablet size and shape on both cylinders. Distance of both cylinders is about 0.2 mm, the openings of both cylinders are identical and cylinders are adjusted in a manner that position of openings is identical which results in formation of symmetrical tablets of round or oval shape. Position of calender is below the front of the die. Rotation speed has to be adjusted to the amount of extruded material in order to achieve a continous process of extrusion and tablet formation.

step 5):"smoothing" of tablets
As distance between cylinders cannot be adjusted to 0, there is a thin (about 0.2 mm) film of extrudat between the tablets. This is removed either by spheronisation (at low speed in order to avoid damage to the tablets) or in a rotating pan.

According to the aforementioned processes, extended release tablets according to the compositions as given in Tables B1 & B2 may be prepared. Release rates depend on thickness of tablets and the composition of excipients (see Fig 8).

### Example F: immediate and extended release matrix tablets produced from melt or wet extrudates

The preparation of the immediate or extended release matrix tablets of the present invention in the following Example usually takes place over the following steps:
step 1): milling of extrudates
step 2): preparation of the main mixture
step 3): preparation of the final mixture
step 4): tabletting
step 5): filling into bottles or preparation of blisters

The equipment described below can be replaced by other pieces which fulfill the same or similar features.

### step 1): preparation and milling of extrudates

Melt extrudates can be used directly after cooling at the end of the running belt, that means without cutting and spheronization (step 3 of example B)
Wet extrudates can be used directly after drying, that means without cutting and spheronization (first part of step 3 of example C)

The extrudates are milled with a Retsch ZM 200 mill sieve size 1.1 mm at 6000 rpm. The resulting powder is sieved using sieves of 100 µm & 700 µm, the fraction from 100 - 700 µm is further processed.

### step 2): preparation of the main mixture

To 200 g of the granules obtained from step 1 the excipients listed in Table F (except Mg-stearate; declared as weight in g) are added and blended for 5 minutes. Subsequently the obtained mixture is sieved (sieve size 0.8 mm).

### step 3): preparation of the final mixture

To the main mixture obtained above in step 2 pre-sieved (sieve size 0.5 mm) magnesium stearate of herbal origin (amount listed in Table G in g) is added and blended for 3 minutes.

### step 4): tabletting

In a suitable tablet pressing apparatus the final mixture as obtained above in step 3 is pressed to obtain the desired tablets. In Process Controls (IPC) are employed as usual.

According to the aforementioned process the following tablets may be prepared: Extrudates produced by examples B or C (excipients as listed in Table B1) are milled as described before. For further processing, excipients as listed in Table F are used.

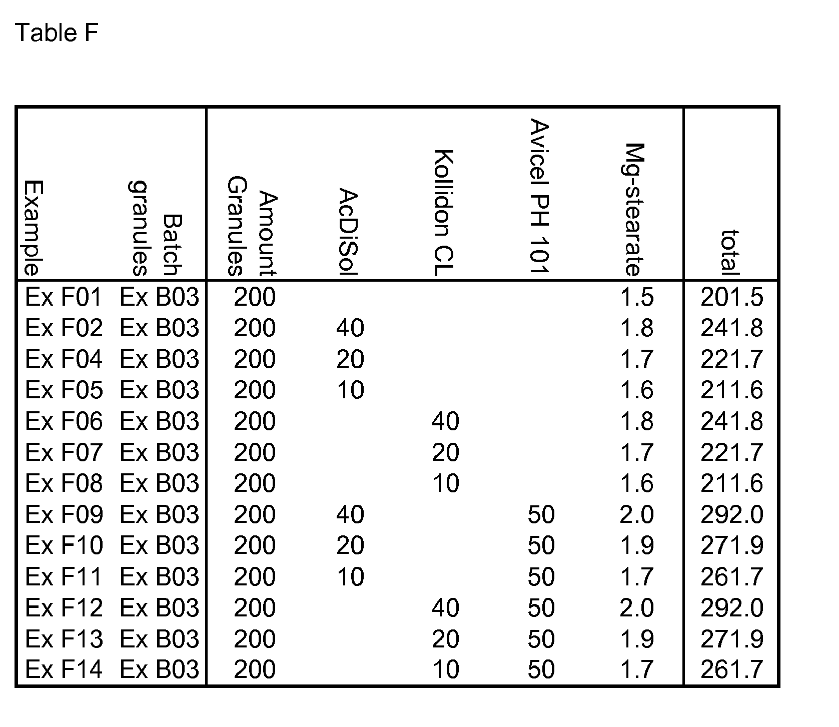

### Example G: immediate or extended release formulations produced by spray drying and subsequent transformation to pellets or tablets

The preparation of the immediate or extended release formulations of the present invention in the following Example usually takes place over 5 steps:
- step 1):: preparation of solution containing active ingredient and one or more acids;
- step 2):: preparation of solution containing supersaturizing excipient(s)
- step 3):: spray drying
- step 4a):: wet or melt extrusion of spray dried material
- step 5a):: filling into capsules
- step 4b):: preparation of tablets using spray dried material.
- step 5b):: filling into bottles or blisters

### step 1): preparation of solution containing active ingredient and one or more acids;

50 g micronized flibanserin and the amount of acid(s) given in Table H1 are dissolved in a mixture of 100 g water, 300 g isopropanole and 600 g tetrahydrofurane under stirring. Then the amount of colloidal siliciium dioxide given in Table H1 is suspended under stirring

### step 2): preparation of solution containing supersaturizing excipient(s)

The amount of supersaturizing excipient(s) given in Table H1 is added slowly to 100 g isopropanole under stirring. When a homogenous suspension is obtained 900 g water are added under stirring and stirred till a homogenous solution is obtained

### step 3): spray drying

immediately before spray drying, the solution from step 2) is added slowly under stirring to the suspension from step 1). Then this solution is pumped with a rate of 2 - 8 g/min (rate is increased over time and adjusted to an outlet air temperature of 65 - 70 °C) into the 0.7 mm nozzle of a Buechi spray drier type 290, inlet air temperature is 140 °C, aspirator efficiency is 75 % of maximum, outlet air temperature is between 65 - 70 °C. After spraying all of the solution heating is switched of and when outlet air temperature is below 40 °C, the spray dryer is stopped and the spray dried material can be sampled from the vessel at the bottom of the cyclone.

### step 4a): wet or melt extrusion of spray dried material

The spray dried powder of step 3 is extruded according to step 3-5 of example B if melt extrusion is employed. In case of wet extrusion processing is according to steps 2) - 4) of example C.

### step 5a): filling into capsules

### Step 5a is identical to step 5) of example B.

### step 4b): preparation of tablets using spray dried material.

To the spray dried material obtained from step 3 the excipients listed in Table G (except Mg-stearate; declared as weight in g) are added and blended for 5 minutes. Subsequently the obtained mixture is sieved (sieve size 0.8 mm). Then pre-sieved (sieve size 0.5 mm) magnesium stearate of herbal origin (amount listed in Table G in g) is added and blended for 3 minutes.
In a suitable tablet pressing apparatus the final mixture as obtained above is pressed to obtain the desired tablets. In Process Controls (IPC) are employed as usual.
The excipients used for spray drying are the same as for listed in Table B1. For further processing, excipients as listed in Table G are used.

According to the aforementioned process the following tablets may be prepared:

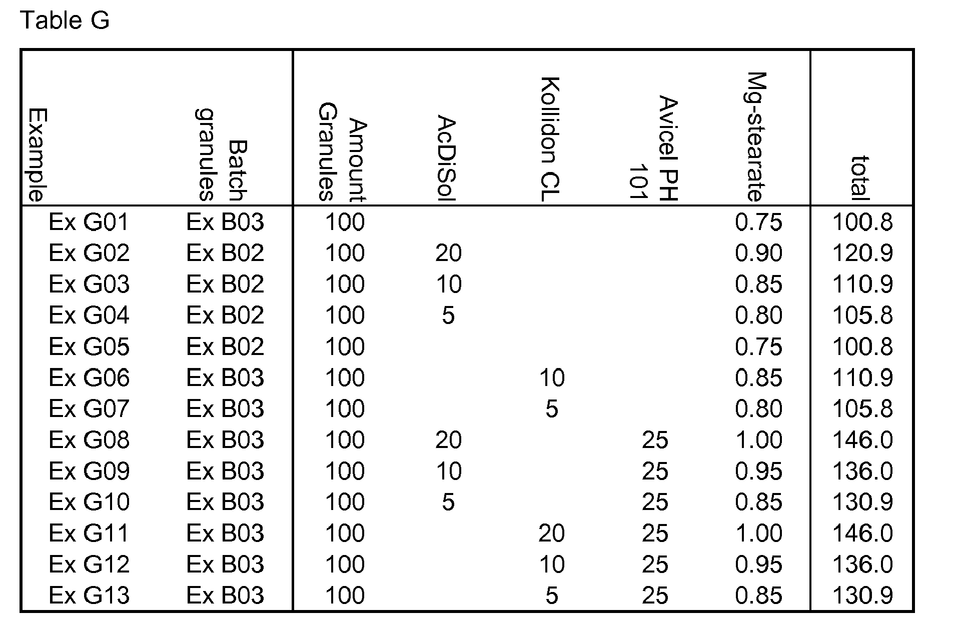

### step 5b): filling into bottles or blisters

Tablets may be filled into blisters or bottles according to methods known to the skilled person.

### Example H: immediate and extended release matrix tablets produced by roller compaction

The preparation of the extended release system of the present invention in the following Example usually takes place over 5 steps:
step 1): preparation of the pre-mixture;
step 2): preparation of the mixture for compaction;
step 3): performing roller compaction;
step 4): preparation of the final mixture; and
step 5): preparation of the tablets.

The steps will be described in the following in detail:
1. Pre-Mixture
   Flibanserin and all excipients listed in Table I except magnesium stearate (numbers indicate grams) are mixed in a usual blender or mixer for 5 minutes.
2. Mixture for Compaction
   To the pre-mixture obtained in above step 1 pre-sieved (sieve size 0.5 mm) magnesium stearate of herbal origin (1/3 of amount listed in Table I) is added and blended in a usual blender or mixer for 3 minutes.
3. Roller Compaction
   The mixture obtained in above step 2 is subjected to a roller compaction process step as known to the skilled in the art.
4. Final Mixture
   To the mixture obtained above in step 3 pre-sieved (sieve size 0.5 mm) magnesium stearate of herbal origin (2/3 of amount listed in Table I) is added and blended for 3 minutes.
5. Tablets

In a suitable tablet pressing apparatus the final mixture as obtained above in step 4 is pressed to obtain the desired tablets. In Process Controls (IPC) are employed as usual.

According to the aforementioned process the following tablets may be prepared:

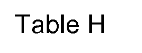

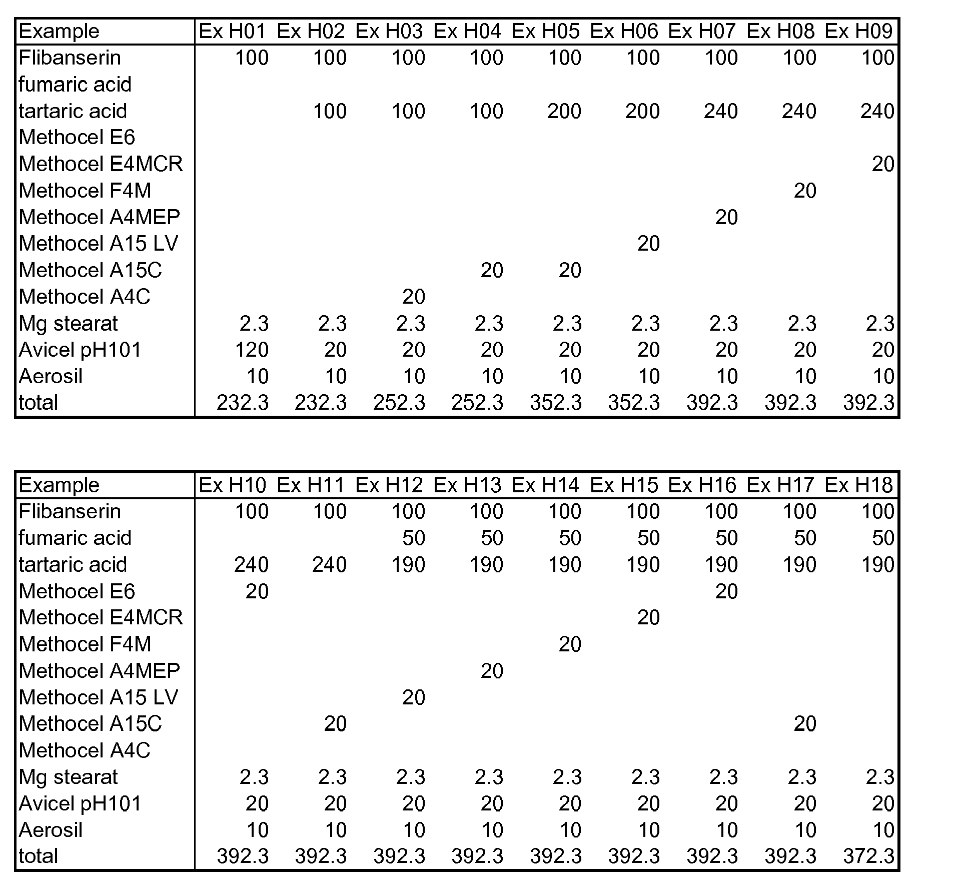

### Example I: immediate and extended release matrix tablets produced by wet granulation

The preparation of the immediate and extended release system of the present invention in the following Example usually takes place over 5 steps:
step 1): preparation of the binder solution
step 2): performing wet granulation;
step 3): drying, milling and sieving
step 4): preparation of the final mixture; and
step 5): preparation of the tablets.

The steps will be described in the following in detail:
1. Pre-Mixture
   Povidone (amount as listed in Table J) is added slowly under stirring into the water (amount as listed in Table J) till complete dissolution is achieved..
2. performing wet granulation
   Flibanserin and all excipients listed in Table I except magnesium stearate and povidone (numbers indicate grams) are mixed in a usual granulation system e.g. a Diosna P1/6 with pot size 1I for 2 minutes with stirrer speed 200 rpm and shredder speed 800 rpm. Then the binder solution is added over 30 " with stirrer speed 200 rpm and shredder speed 800 rpm and stirring is continued until an increase in currency indicates that granulation is complete.
3. drying, milling and sieving
   The granulate obtained in above step 2 is dried, milled and sieved with procedures as known to the skilled in the art.
4. Final Mixture
   To the granules obtained above in step 3 pre-sieved (sieve size 0.5 mm) magnesium stearate of herbal origin (amount as listed in Table J) is added and blended for 3 minutes.
5. Tablets
   In a suitable tablet pressing apparatus the final mixture as obtained above in step 4 is pressed to obtain the desired tablets. In Process Controls (IPC) are employed as usual.

According to the aforementioned process tablets according to compositions as listed in Table H may be prepared. In addition to excipients of Table H, a binder e.g. povidone K 25 (amount is 8 % of total mass except Mg-stearate) is dissolved in appropriate amount of water and used as binder solution for wet granulation

### Example J: immediate and extended release pellets produced by pellet layering

The preparation of the immediate and extended release system of the present invention in the following Example usually takes place over 5 steps:
step 1): preparation of core material containing pH modifier;
step 2): preparation of the first insulating layer;
step 3): preparation of the second layer containing active substance and supersaturizer;
step 4): preparation of the third layer;
step 5): packing into capsules.

### The steps will be described in the following in detail:

### Step 1) Preparation of Core Material Containing pH Modifier

Core materials can be produced by wet or melt extrusion of different acids or mixtures thereof or layering of powdered acids onto spherical acid crystals by methods known to those by skilled in the art.

### Step 2) Isolation of the Core Material Containing Acid

0.5 parts of hypromellose are dissolved in 10.1 parts of 96 % ethanol. Further 0.5 parts of talc together with 0.01 parts of polydimethylsiloxane are dispersed into the hypromellose/ethanol solution with stirring. This insulating dispersion is sprayed onto the acid cores (step1) in a fluidised bed processing plant, 21 parts by weight of tartaric acid-containing core material are sprayed with the hypromellose/talc dispersion at an air entry temperature of 35°-40° C. by the under-bed spraying method. The isolated tartaric acid-containing core material is then dried in the circulating air dryer at 40° C for 8 hours. To remove lumps the dried isolated acid-containing core material is screened through a screen with a nominal mesh size of 1.0 mm. The fraction of material (particle size less than 1 mm) is further processed.

### Step 3) Preparation of the Second Layer Containing the Active Substance and supersaturizer;

### 1. Preparation of the Lake Solution

Isopropyl alcohol (amount as indicated in Table J) was charged in a suitable reaction vessel and then povidone (binder; amount as indicated in Table J), is added in portions under stirring. When complete dissolution is achieved, flibanserin, supersaturizing agent and talc (amounts as indicated in Table J) were dispersed in this solution with stirring. The solution was stirred at room temperature overnight. It was obtained a lake solution.

### 2. Spraying of the obtained lake solution

Then the lake solution was sprayed onto the product obtained in step 2). To this purpose the product was placed in a suitable coating apparatus (e.g. a Huettlin Microlab fitted with an air inlet and exhaust. At an air inlet temperature of about 25°C the product was sprayed with the lake solution in continuous operation and sprinkled so as to produce roughly spherical particles. The following conditions were used:

| | |
|---|---|
| inlet air quantity | 12 m³/h |
| spraying rate | 0.5 - 2 g/min |
| spray pressure | 0.6 bar, |
| micro climate | 0.3 bar |
| nozzle diameter | 0.6 mm |
| spray time | about 2 h |
| product temperature | 30 - 35°C |

The virtually spherical product obtained was then dried in a suitable drying device at 40°C for 12 hours. The product was fractionated using a suitable screening machine with perforated plates having nominal mesh sizes of 1.25 mm.

Step 4a) Preparation of the Lake Solution for pH-dependent polymers Isopropyl alcohol and acetone were charged in a suitable reaction vessel then Eudragit S100 and hypromellosephathalat 55 were added in portions and dispersed in this solution with stirring, till complete dissolution occurred. Then water and triethyl citrate were added slowly in order to maintain a clear solution. Then talc was added under stirring. The amounts of solvents and excipients are given as g in Table C. The solution was stirred continously also during spraying

Step 4b) Preparation of the Lake Solution for pH-independent polymers Isopropyl alcohol and water were charged in a suitable reaction vessel then Ethylcellulose and PEG 6000 were added in portions and dispersed in this solution with stirring, till complete dissolution occurred. Then talc was added under stirring. The amounts of solvents and excipients are given as g in Table C. The solution was stirred continously also during spraying

### Step 4c) Spraying of the obtained lake solution

The lake solution obtained from step 4a) or 4b) was sprayed onto the melt extrusion pellets (amount of pellets is given as g in Table J). To this purpose the pellets were placed in a suitable Huettlin Microlab fitted with an air inlet and exhaust. At an air inlet temperature of about 45°C the product was sprayed with the lake solution in continuous operation and sprinkled so as to produce roughly spherical particles. The following conditions were used:

| | |
|---|---|
| inlet air quantity | 12 m³/h preferably |
| spraying rate | 0.3 - 1.5 g/min (rate is increased gradually during spraying) |
| spray pressure | 0.6 bar, |
| micro climate | 0.3 bar |
| nozzle diameter | 0,8 mm |
| spray time | about 1.5 h |
| product temperature | 30 - 35°C |

The virtually spherical product obtained was then dried in a suitable drying device at 40°C for 12 hours. The product was fractionated using a suitable screening machine with perforated plates having nominal mesh sizes of 0.8 and 1.25 mm, the fraction between 0.8 and 1.25 mm was used for capsule filling.

According to the aforementioned processes the following pellets may be prepared:

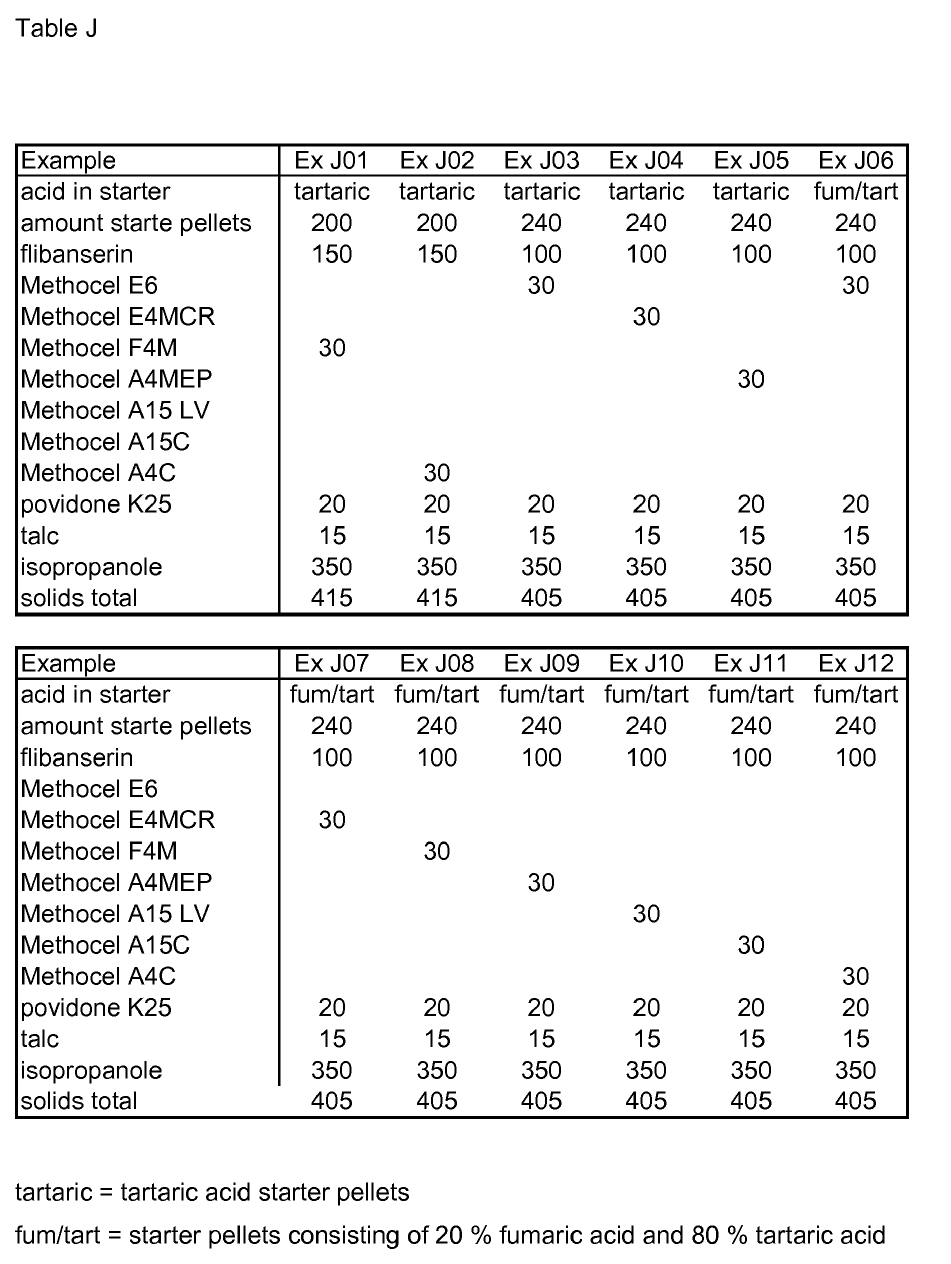

The virtually spherical product obtained was then dried in a suitable drying device at 40°C for 12 hours. The product was fractionated using a suitable screening machine with perforated plates having nominal mesh sizes of 1.25 mm.

## Claims

1. Pharmaceutical release system comprising or essentially consisting of
I) a core comprising
a) Flibanserin or a pharmaceutically acceptable derivative thereof as active ingredient;
b) one or more supersaturizing excipient(s) selected from the group consisting of methylcelluloses with nominal viscosity of 400, methylcelluloses with nominal viscosity of 1500, methylcelluloses with nominal viscosity of 400 cP, methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP;
c) one or more pharmaceutically acceptable pH modifiers in a weight ratio of Flibanserin : pH modifiers of 2 : 1 or lower; and
d) optionally one or more additives;
and in case of an extended release system
e) optionally one or more retarding agents; and/or
II) one more retard layers comprising one or more retarding agents surrounding, but not necessarily in direct contact with the active ingredient.

2. Pharmaceutical release system comprising or essentially consisting of
I) a core comprising
a) Flibanserin or a pharmaceutically acceptable derivative thereof as active ingredient;
b) one or more supersaturizing excipient(s) selected from the group consisting of methylcelluloses, hypromellose 2208, hypromellose 2910 and hypromellose 2906;
c) one or more pharmaceutically acceptable pH modifiers, wherein the pH modifiers are present in an amount of 45 - 90 % by weight of the core;
d) optionally one or more additives;
and in case of an extended release system
e) optionally one or more retarding agents; and/or
II) one more retard layers comprising one or more retarding agents surrounding, but not necessarily in direct contact with the active ingredient.

3. Pharmaceutical release system according to claim 2 wherein the one or more supersaturizing excipient(s) are selected from the group consisting of methylcelluloses with nominal viscosity of 15 cP, methylcelluloses with nominal viscosity of 400 cP, , methylcelluloses with nominal viscosity of 1500 cP, methylcelluloses with nominal viscosity of 4000 cP, hypromellose 2208 with nominal viscosity of 4000 cP, hypromellose 2208 with nominal viscosity of 15000 cP, hypromellose 2910 with nominal viscosity of 3 cP, hypromellose 2910 with nominal viscosity of 5 cP, hypromellose 2910 with nominal viscosity of 6 cP, hypromellose 2910 with nominal viscosity of 15 cP, hypromellose 2910 with nominal viscosity of 50 cP, hypromellose 2910 with nominal viscosity of 4000 cP, hypromellose 2906 with nominal viscosity of 50 cP and hypromellose 2906 with nominal viscosity of 4000 cP.

4. Pharmaceutical release system according to one or more of the claims 1 to 4 **characterized in that** the amount of the supersaturizing excipient(s) is between 0.3 - 40 % by weight of the core.

5. Pharmaceutical release system according to one or more of the claims 1 to 3 **characterized in that** the amount of the supersaturizing excipient(s) is between 0.6 - 20 % by weight of the core.

6. Pharmaceutical release system according to one or more of the claims 1 to 3 **characterized in that** the amount of the supersaturizing excipient(s) is between 1 - 15 % by weight of the core.

7. Pharmaceutical release system according to one or more of the claims 1 to 3 **characterized in that** the amount of the supersaturizing excipient(s) is between 2 - 10 % by weight of the core.

8. Pharmaceutical release system according to one or more of the claims 2 to 7 **characterized in that** the amount of the one or more pH modifier is between 50 - 80 % by weight of the core.

9. Pharmaceutical release system according to one or more of the claims 2 to 7 **characterized in that** the amount of the one or more pH modifier is between 57 - 77 % by weight of the core.

10. Pharmaceutical release system according to one or more of the claims 2 to 7 **characterized in that** the amount of the one or more pH modifier is between 58 - 72 % by weight of the core.

11. Pharmaceutical release system according to one or more of the claims 1 to 10 **characterized in that** the one or more pH modifier is selected from the group consisting of adipic acid, ascorbic acid, aspartic acid, citric acid, fumaric acid, lactic acid, malic acid, succinic acid and tartaric acid, more preferred are succinic acid, tartaric acid, lactic acid and fumaric acid.

12. Pharmaceutical release system according to one or more of the claims 1 to 10 **characterized in that** the one or more pH modifier is selected from the group consisting of a combination of a) tartaric acid and lactic acid, b) tartaric acid and fumaric acid, c) tartaric acid, lactic acid and fumaric acid.

13. Use of a pharmaceutical release system according to one or more of claims 1 to 12 for the manufacture of a medicament for the treatment of central nervous system disorders, affective disorders, anxiety, sleep and sexual disorders (Hypoactive Sexual Desire Disorder, premenstrual disorders like premenstrual dysphoria, premenstrual syndrome, premenstrual dysphoric disorder; sexual aversion disorder, sexual arousal disorder, orgasmic disorder, sexual pain disorders like dyspareunia, vaginismus, noncoital sexual pain disorder; sexual dysfunction due to a general medical condition and substance-induced sexual dysfunction), psychosis, schizophrenia, personality disorders, mental organic disorders, mental disorders in childhood, aggressiveness, age associated memory impairment, neuroprotection, neurodegenerative diseases, cerebral ischaemia of various origins, anorexia nervosa, Attention Deficit Hyperactivity Disorder (ADHD), obesity, urinary incontinence, chronic pain and Valvular Heart Disease.

14. Use according to claim 13, **characterized in that** flibanserin is applied in form of a polymorph A of the free base, having a melting point of about 161 °C as measured using DSC.

15. Use according to one or more of the preceding claims, **characterized in that** flibanserin is applied in a dosage range between 0.1 to 400 mg per day.

16. Use according to one or more of the preceding claims, **characterized in that** flibanserin is applied once or twice daily consecutively over a period of time.

17. Use according to one or more of the preceding claims, **characterized in that** flibanserin is applied in the morning and the evening.

18. Use according to one or more of the preceding claims, **characterized in that** flibanserin is applied once in the evening only (50 or 100 mg of flibanserin) consecutively over a period of time.

19. Method for manufacturing a pharmaceutical release system according to one or more claims 1 to 12 **characterized in that** it is the system is manufactured by extrusion.

20. Method according to claim 14 **characterized in that** no microcrystalline cellulose is used during the manufacturing process.

21. Method according to claim 14 or 15 **characterized in that** no plasticizer is used during the manufacturing process.

22. Method for manufacturing a pharmaceutical release system according to one or more claims 1 to 12 **characterized in that** it is the system is manufactured by spray drying.

23. Method according to claim 17 **characterized in that** no microcrystalline cellulose is used during the manufacturing process.
